# EUROPEAN PATENT SPECIFICATION

(11) **EP 3 487 537 B1**
(45) Date of publication and mention of the grant of the patent: **16.10.2024**
(21) Application number: 17831902.6
(22) Date of filing: 20.07.2017
(51) Int. Cl.: A01N 1/02, A01N 25/04, A61K 31/704, A61K 35/19, A61K 8/04, A61L 27/34, A61L 27/52, A61L 27/54, A61Q 19/00, A61K 47/30

(54) **ZWITTERIONIC MICROGELS, THEIR ASSEMBLIES AND RELATED FORMULATIONS, AND METHODS FOR THEIR USE**
ZWITTERIONISCHE MIKROGELE, DEREN ANORDNUNGEN UND ZUGEHÖRIGE FORMULIERUNGEN UND VERFAHREN ZU DEREN VERWENDUNG
MICROGELS ZWITTÉRIONIQUES, LEURS ENSEMBLES ET FORMULATIONS ASSOCIÉES, ET LEURS PROCÉDÉS D'UTILISATION

(30) Priority: 22.07.2016 US 201662365788 P
(43) Date of publication of application: 29.05.2019
(73) Proprietor: University of Washington, Seattle, WA 98105-4608 (US)
(72) Inventor: JIANG, Shaoyi, Seattle WA 98105-4608 (US); SINCLAIR, Andrew, Seattle WA 98105-4608 (US); O'KELLY, Mary, Elizabeth, Seattle WA 98105-4608 (US); BAI, Tao, Seattle WA 98105-4608 (US); JAIN, Priyesh, Seattle WA 98105-4608 (US)
(74) Representative: MacLean, Martin Robert
(86) International application number: PCT/US2017/043153
(87) International publication number: WO 2018/017879

(56) References cited:
- WO-A1-2016/040489
- US-A1- 2004 208 985
- US-A1- 2011 195 104
- No further relevant documents disclosed

## Description

### BACKGROUND OF THE INVENTION

Hydrogels are hydrated elastic polymer networks that share many properties with natural tissues. Many of their growing biomedical applications, including cosmetic procedures, localized therapeutic delivery and as regenerative cell scaffolds, demand injectability or malleability to avoid invasive surgery or fill unique three-dimensional (3 D) volumes. Among the natural and synthetic polymers used to construct hydrogels, polyzwitterions have gained particular attention in recent years because of their uniquely biocompatible attributes. These polymers contain repeated pairs of cationic and anionic groups along their chain, mimicking the phospholipids comprising cell membranes or the mixed-charge surfaces of many proteins. Zwitterionic polymer brushes, hydrogels, and elastomers confer ultra-low levels of nonspecific protein fouling from complex physiological fluids, exceeding the performance of popular hydrophilic or amphiphilic polymers like poly(ethylene glycol) (PEG). Hydrogels formed from pure zwitterionic polycarboxybetaine (PCB) and CB crosslinkers can inhibit the foreign body response and resist collagenous capsule formation when implanted in mice, as well as shield proteins from immunogenic responses in the bloodstream. Key to regenerative medicine applications, stem cells encapsulated in PCB hydrogels maintain their therapeutic multipotency and avoid nonspecific differentiation. While zwitterionic hydrogels surpass the biocompatibility, physiological stability, and non-immunogenicity of those based on polysaccharides and PEG, no straightforward route to injectable or malleable pure zwitterionic hydrogels has been reported to date. In addition, most reported zwitterionic 3-D cell culture systems require chemistries or techniques that would be difficult to implement in a clinical lab or biomanufacturing setting. As zwitterionic hydrogels move towards clinical use, these dynamic material properties and ease-of-use are increasingly important.

Making a hydrogel injectable (able to pass through a needle) or malleable (able to be molded into new shapes without cracking), while also maintaining its tissue-like elasticity is difficult, and creative crosslinking strategies have been developed to address this challenge. One class of injectable hydrogel, frequently described as *in situ forming,* leverages bioorthogonal 'click' reactions to spontaneously form a covalently crosslinked network when two components are mixed or injected together. For example, thiol-ene coupling and azide-alkyne cycloaddition reactions such as SPAAC have both been used to develop *in situ*-forming PEG-based hydrogels, such as described in DeForest and Anseth, Nature Chemistry, 3, 2011, 925. This crosslinking strategy is useful for 3-D cell encapsulation, as it avoids radical-based chain reactions that can damage cells, leave behind toxic molecules, and are difficult to initiate *in vivo.* However, reminiscent of epoxy glues, *in situ* network formation is typically irreversible and the gels cannot be significantly re-shaped once formed. Additionally, these polymer architectures are often expensive and complex to develop, and require significant optimization for different applications. Many of these gels are based on PEG because of its presumed biocompatibility, but PEG has been increasingly reported to cause immunogenic reactions.

A second class of dynamic or injectable hydrogel relies on some form of physical crosslinking, which can enable repeated switching between "solid-like" and "liquid-like" forms under different conditions. For many clinical applications, these materials are more practical and useful than irreversible *in situ* forming gels. Some of these can be thermally triggered to reversibly assemble into physically crosslinked supramolecular structures, such as NIPAM block copolymers or PEG-based Pluronics/poloxamers. While these are commonly used for injectable drug formulations, their lack of covalent crosslinking makes them relatively weak and short-lived *in vivo,* their temperature sensitivity requires refrigerated storage, and many variations result in toxicity as they disassemble. Other reversible gels are often referred to as viscoelastic hydrogels: these are commonly designed to flow in response to increased shear (such as when pushed through a needle) and then self-heal into a new elastic shape. Many gels in this category are based on polysaccharides, such as alginate, dextran, and hyaluronic acid; these natural polymers can reversibly crosslink by chelating divalent ions such as Ca²⁺ and Mg²⁺. However, there are many inherent limitations of polysaccharide gels, which have poor long-term physiological stability, varying biocompatibility, and are difficult and costly to purify from natural sources or synthesize into medical grade materials. US2004208985A1 describes local drug delivery. WO2016040489A1 describes functionalized zwitterionic and mixed charge polymers, related hydrogels, and methods for their use. US2011195104A1 describes integrated antimicrobial and low fouling materials.

Despite the advances in injectable hydrogels noted above, a need exists for improved injectable hydrogels and versatile cell scaffolds targeting practical clinical needs. The present invention seeks to fulfill this need and provides further related advantages.

### SUMMARY OF THE INVENTION

The present invention is defined by the claims. This disclosure provides injectable and malleable hydrogels combining high biocompatibility, physiological stability and ease-of-use that are highly desirable for biomedical applications. This disclosure provides self-healing zwitterionic and mixed charge microgels. Zwitterionic polycarboxybetaine (PCB) forms superhydrophilic and non-immunogenic hydrogels completely devoid of nonspecific cell and tissue interactions, uniquely enabling PCB to mitigate the foreign body reaction. This disclosure provides a simple and scalable strategy to create injectable self-healing zwitterionic and mixed charge hydrogels and cell scaffolds by reconstructing microgel units into new bulk materials. The combination of covalent crosslinking inside each microgel and supramolecular interactions between them gives the resulting zwitterionic injectable pellet (ZIP) constructs supportive moduli and tunable viscoelasticity. Lyophilized ZIP powders retain their strength and elasticity upon rehydration, simplifying sterilization and storage. When reconstituted with any aqueous solution or suspension containing cells, proteins, or drug-loaded microspheres, ZIP powders rapidly self-heal into a homogeneous composite hydrogel formulation without any specialized reagents or conditions. These materials are useful as highly biocompatible tissue fillers, protective cell scaffolds, and broadly applicable carriers for injectable therapies. The appended claims are directed to methods of the invention, and describe microgels used within the claimed methods.

In view of the foregoing, in one aspect the disclosure provides a method of claim 1.

In another aspect, the disclosure provides a method for cell culturing as defined in the claims. The method comprises culturing a population of cells in a matrix comprising a zwitterionic microgel, as defined in the claims.

In another aspect, the disclosure provides a method for protectively storing a population of cells, a tissue, or an organ, comprising storing a population of cells, a tissue, or an organ as defined in the claims. The methodcomprises a zwitterionicmicrogel as defined in the claimsto provide stored cells, a stored tissue or a stored organ, wherein the stored cells, stored tissue, or stored organ substantially retains its biological function on storage.

The disclosure provides a microgel composition prepared from physical processing of a crosslinked zwitterionic hydrogel or a crosslinked mixed charge hydrogel to provide a microgel composition comprising a plurality of crosslinked zwitterionic or a plurality of crosslinked mixed charge microgel units, respectively.

### DESCRIPTION OF THE DRAWINGS

The foregoing aspects and many of the attendant advantages of this invention will become more readily appreciated as the same become better understood by reference to the following detailed description, when taken in conjunction with the accompanying drawings.

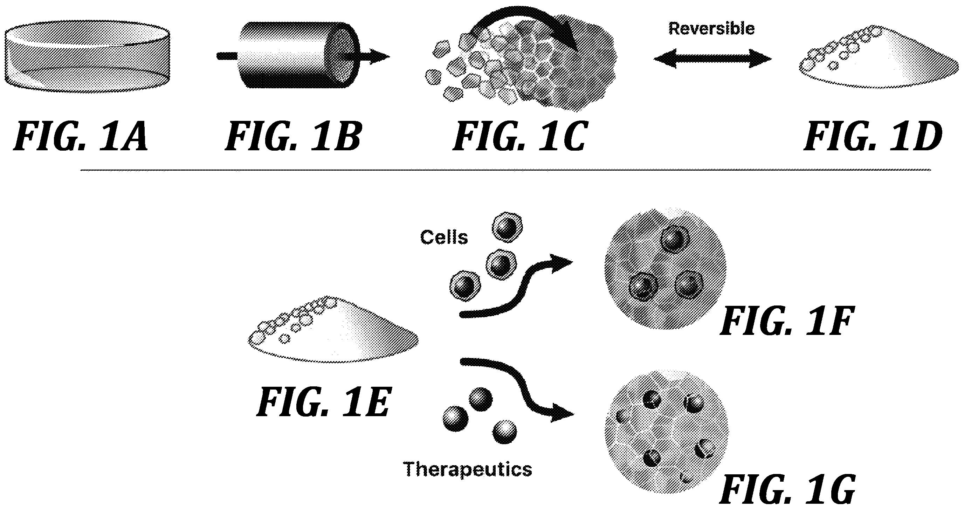

FIGURES 1A-1F schematically illustrate the preparation, properties, and usefulness of representative zwitterionic microgels . FIGURES 1A-1D present an overview schematic showing the production of representative viscoelastic zwitterionic injectable pellet (ZIP) gels , which can be reversibly lyophilized for simple formulations. FIGURES 1E-1F show representative examples of formulations created by reconstituting lyophilized microgels with cells of therapeutics.

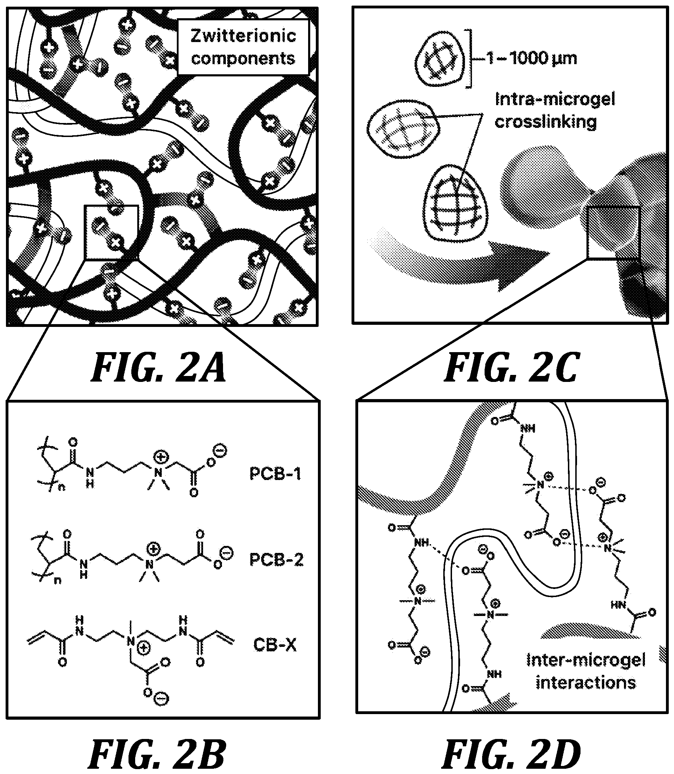

FIGURES 2A-2D show representative hydrogel components that are purely zwitterionic, consisting of carboxybetaine acrylamide monomers (CB-1 or CB-2) with carboxybetaine diacrylamide crosslinker (CB-X). FIGURE 2B depicts the chemical structures of PCB-1, PCB-2, and CB-X. FIGURE 2C illustrates covalent crosslinks inside each microgel that enable bulk elasticity. FIGURE 2D illustrates zwitterionic fusion consisting of dynamic interactions that reconstruct microgels into a new viscoelastic material.

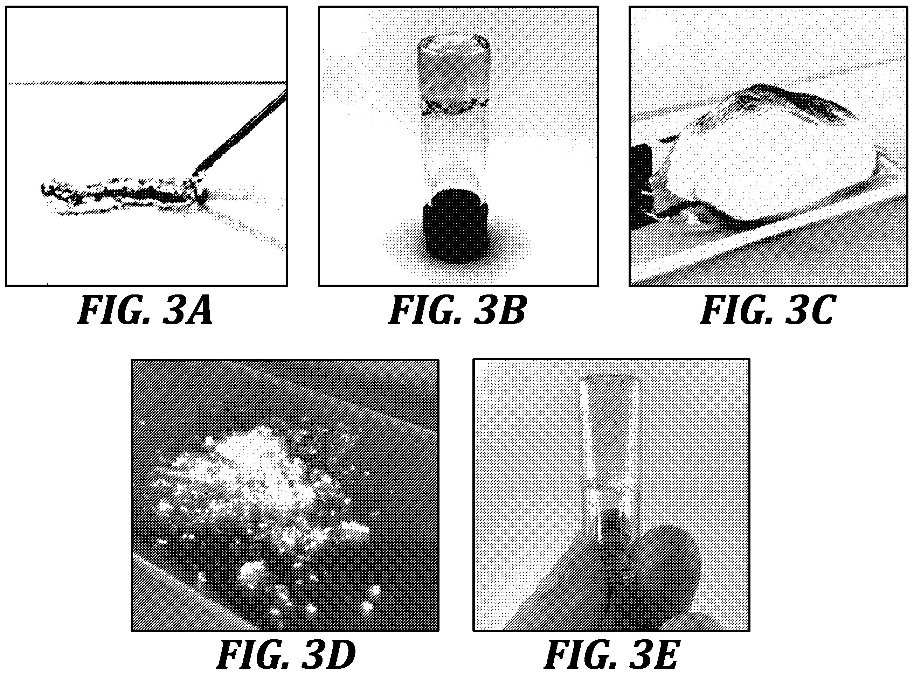

FIGURES 3A-3E are photographs illustrating representative properties of microgel materials. FIGURE 3A illustrates injectability. FIGURE 3B illustrates self-healing in a vial. FIGURE 3C illustrations self-supporting properties of the microgel composition. FIGURE 3D illustrates a lyophilized microgel composition. FIGURE 3E illustrates that once reconstituted, microspheres remained suspended in microgel formulation indefinitely (4 weeks or more without visible settling) demonstrating stable injectable formulations.

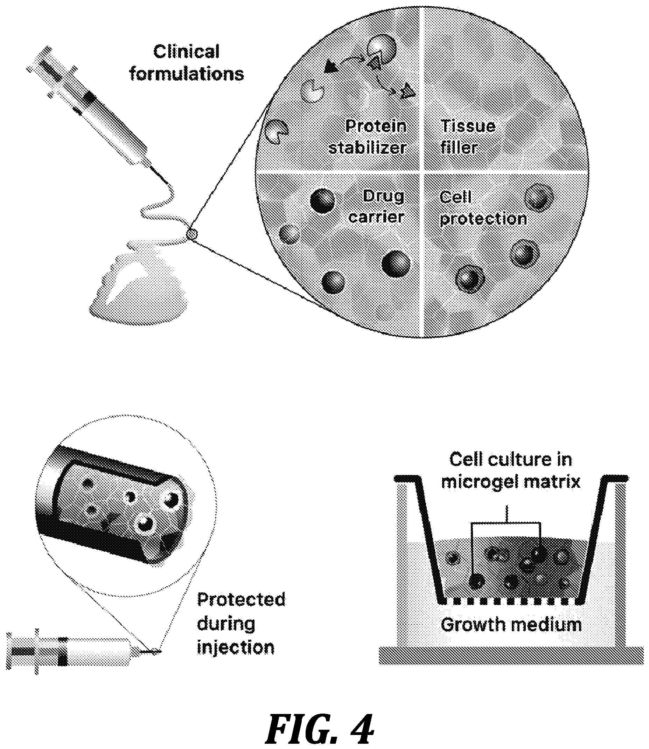

FIGURE 4 illustrates representative applications of microgel compositions: as injectable soft tissue fillers, therapeutic carriers and cell scaffolds for growth and injection. Bottom left: illustrates injectable scaffold made from ZIP gel. Bottom right: Transwell *in vitro* cell culture setup with porous membrane for media equilibration, used to grow and preserve CD4+ T cells.

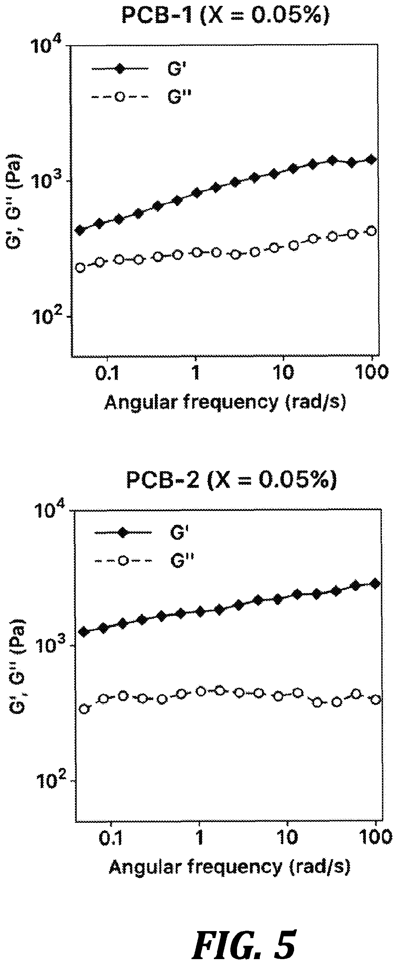

FIGURE 5 compares selected dynamic oscillatory frequency experiments of representative CB-1 and CB-2 based ZIP hydrogels of the invention (same crosslinker content, X = 0.05%). G' is dominant over G" at all frequencies, showing the elastic network remains in place under a wide range of conditions.

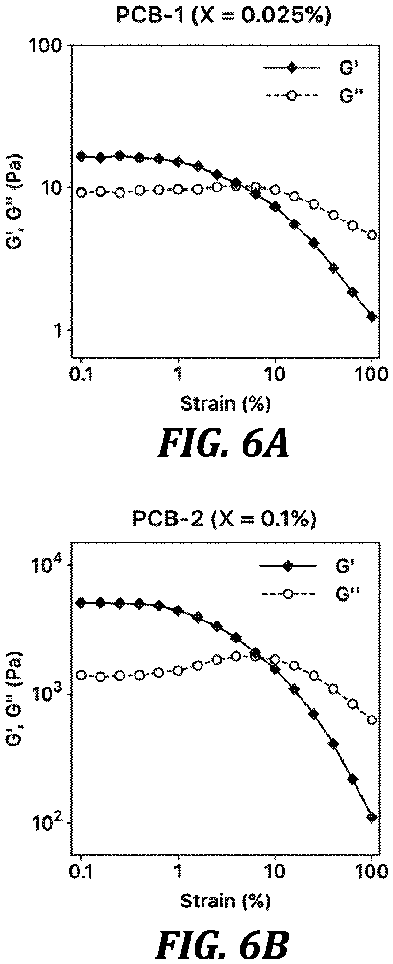

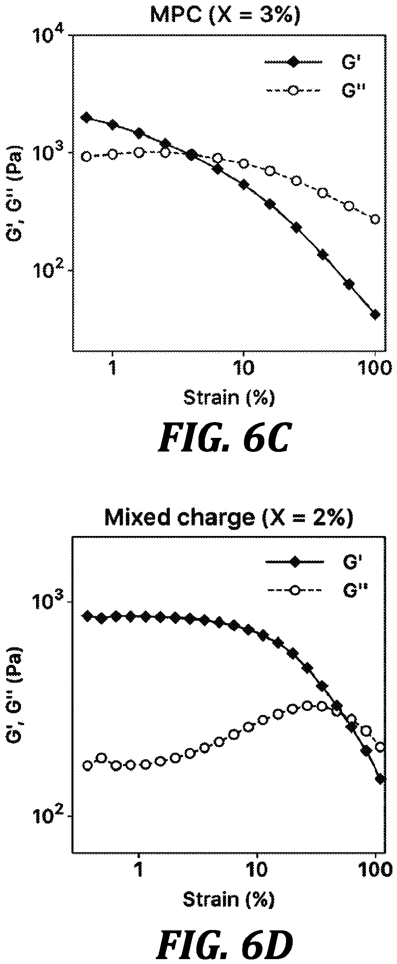

FIGURES 6A-6D compare shear-thinning rheological properties of representative microgel compositions of the invention as measured by oscillatory strain sweep tests: PCB-1 based zwitterionic microgels, PCB-2 based zwitterionic microgels, MPC based zwitterionic microgels, and mixed charge microgels. In all subfigures, storage (G', solid markers) and loss (G", open markers) moduli are plotted as strain increases from <1% to 100%. FIGURE 6A illustrates a PCB-1 based sample with lower crosslinking (0.025% CB-X). FIGURE 6B illustrates a PCB-2 based sample with higher crosslinking (0.1% CB-X). FIGURE 6C illustrates an MPC based sample 3% crosslinking. FIGURE 6D illustrates a mixed charge sample with 2% crosslinking.

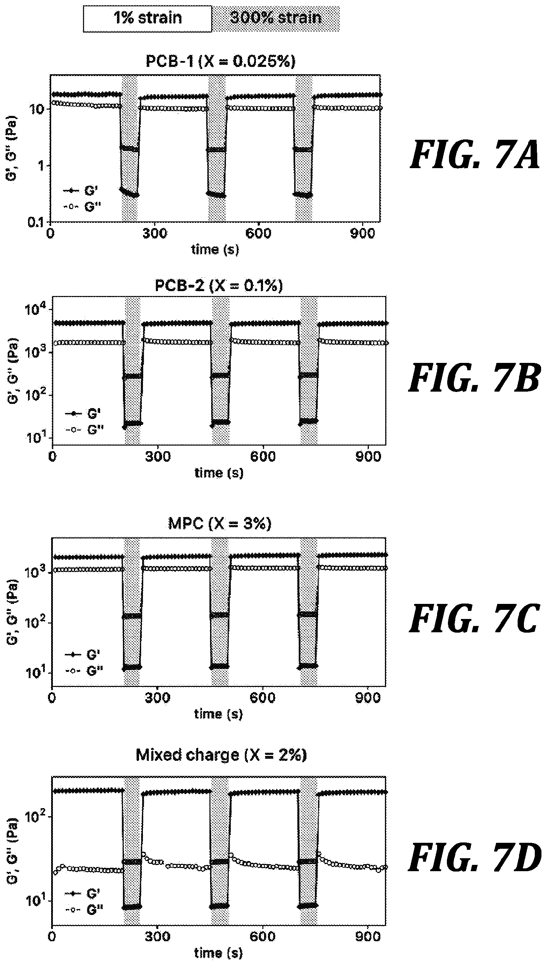

FIGURES 7A-7D compare self-healing properties of representative microgel formulations as measured by rheological step-strain tests. In all subfigures, storage (G', solid markers) and loss (G", open markers) moduli are plotted as the strain is toggled between 1% (white background) and 300% (shaded background). FIGURE 7A illustrates a PCB-1 based sample with lower crosslinking (0.025% CB-X). FIGURE 7B illustrates a PCB-2 based sample with higher crosslinking (0.1% CB-X). FIGURE 7C illustrates an MPC based sample with 3% crosslinking. FIGURE 7D illustrates a mixed charge sample with 2% crosslinking.

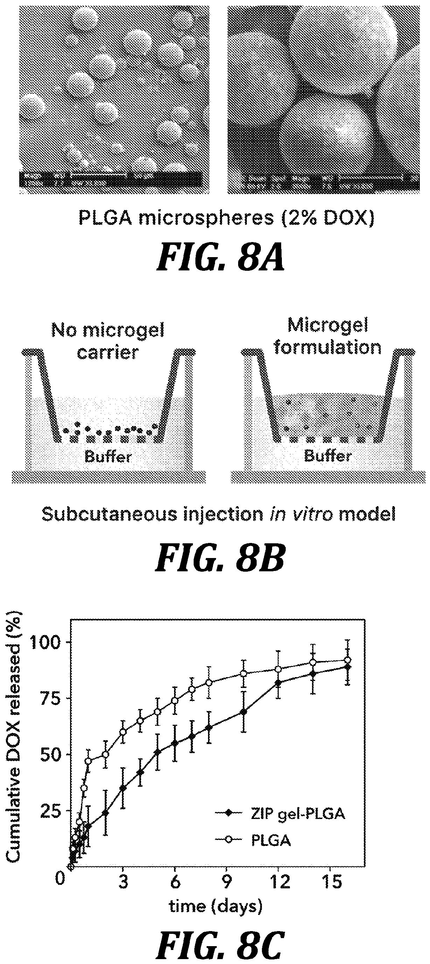

FIGURES 8A-8C (comparative example) illustrate zwitterionic microgels as carriers for PLGA-encapsulated drugs. FIGURE 8A illustrates SEM micrographs of PLGA microspheres loaded with doxorubicin (DOX) at two magnifications (1200x and 3500x). FIGURE 8B schematically illustrates the *in vitro* model used to measure DOX release rate. FIGURE 8C compares DOX release over two weeks *in vitro* from PLGA MS (open circles) and PLGA MS suspended in ZIP gel (solid diamonds).

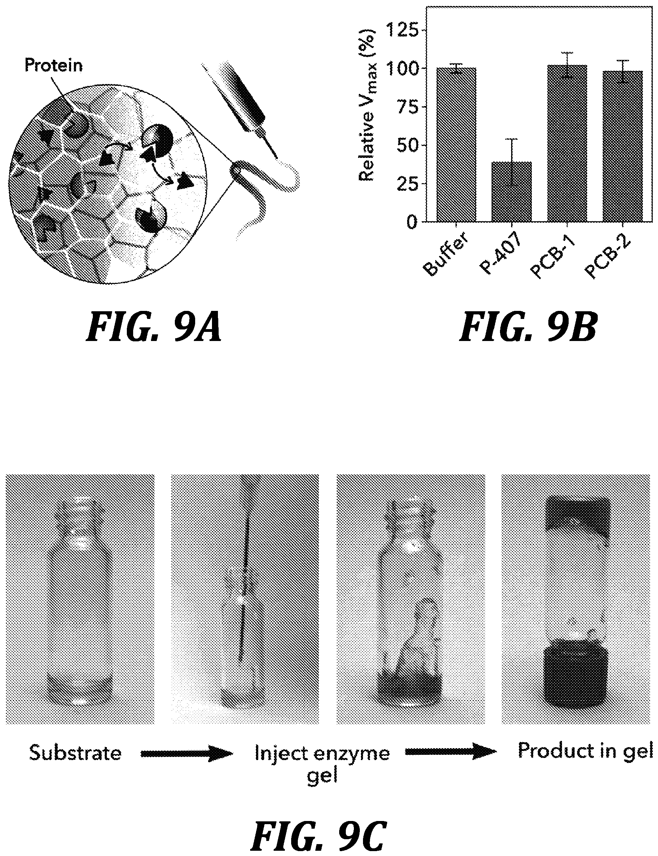

FIGURE 9A (comparative example) schematically illustrates an active enzyme gel (e.g., for topical or injectable biologic delivery applications). FIGURE 9B (comparative example) compares the kinetic evaluation of active enzyme gels formulated with representative zwitterionic microgels of the invention (PCB-1 or PCB-2) and β-Lactamase (B-La), showing Vₘₐₓ equivalent to B-La in buffer; Pluronic^{®}-based injectable enzyme gel (P-407; PEG-PPG-PEG triblock) significantly reduced activity. FIGURE 9C are images of a B-La loaded ZIP gel injected over nitrocefin substrate, which rapidly catalyzed substrate conversion inside the gel.

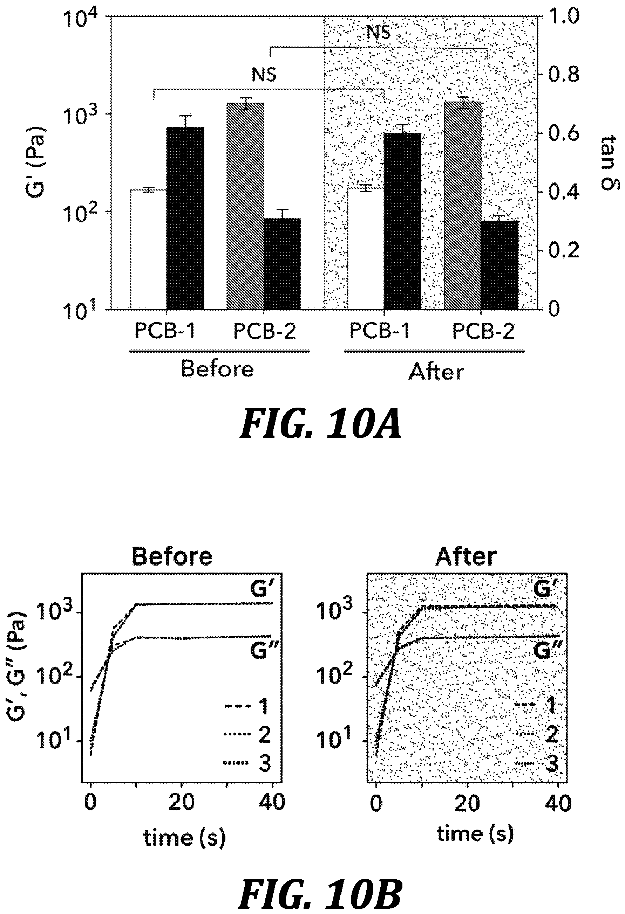

FIGURE 10A compares strength (G', left axis, light and shaded bars) and elasticity (tangent δ, right axis, black bars) of PCB-1 (light bars) and PCB-2 (shaded bars) ZIP gels before and after lyophilization (mean ± s.e.m.); both gels contain 0.05% CB-X. Post lyophilization gels were rehydrated to their equilibrium water content (EWC). FIGURE 10B compares recovery of G' and G" by CB-2 (CB-X = 0.05%) ZIP gels upon reverting from high (300%) to low (1%) strain after step-strain cycles 1-3: prior to lyophilization (left); post-lyophilization and rehydration to EWC (right).

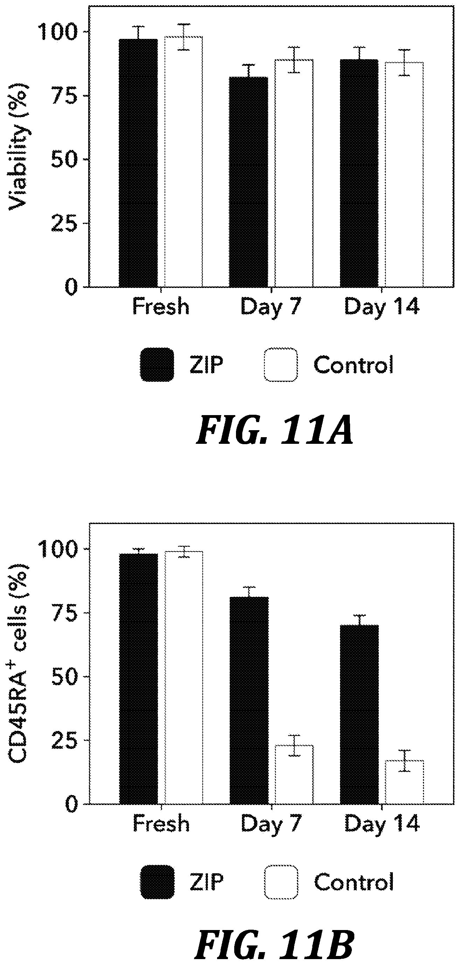

FIGURES 11A-11B illustrate three-dimensional (3-D) T cell growth and preservation in representative reconstituted ZIP hydrogels of the invention. FIGURE 1 1A compares viability of T cells in ZIP gels and control cultures after 7 and 14 days. FIGURE 11B compares CD45RA expression by fresh T-cells, populations cultured in ZIP gels, and control cultures, after 7 and 14 days.

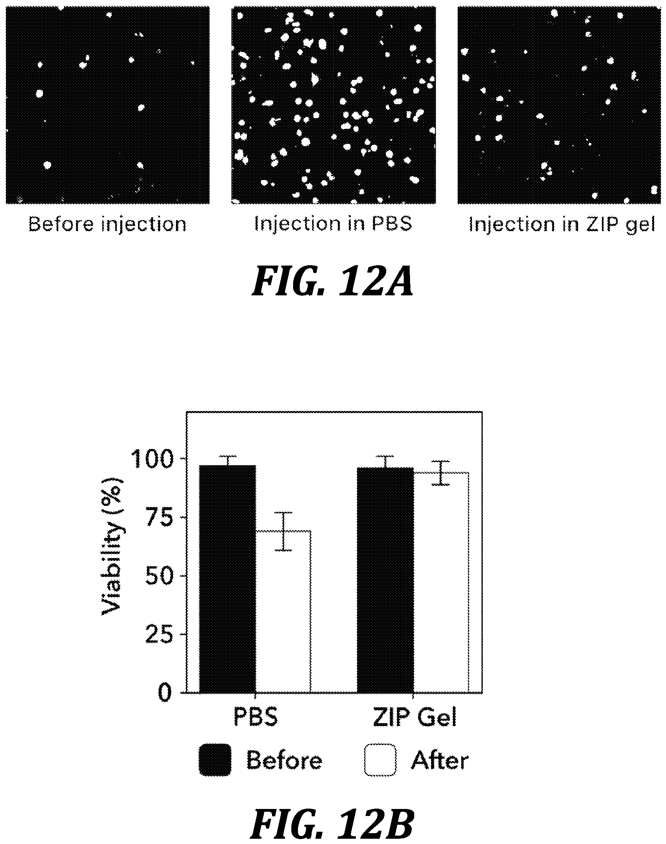

FIGURE 12A compares LIVE/DEAD stained cells, using HEK 293 cells as model cell line, before and after injection through a 28-G needle in phosphate buffered saline (PBS) and ZIP gel formulations (dead cells shown). FIGURE 12B compares viability before and after injection for phosphate buffered saline (PBS) and ZIP gel formulations.

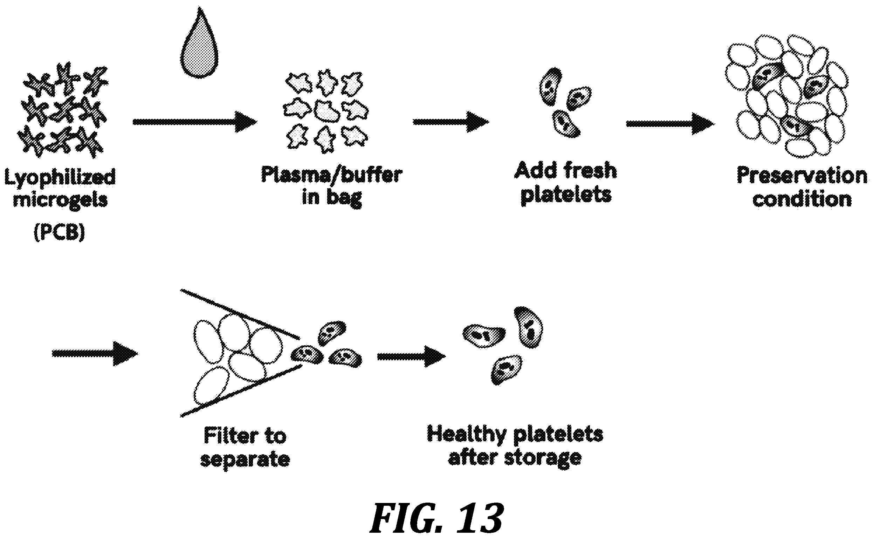

FIGURE 13 is a schematic illustration of a zwitterionic microgel platelet preservation strategy: fresh platelets in plasma were added to lyophilized microgels in a platelet storage bag, with platelets suspended and supported by the PCB microgels (comingled storage), but not interacting with the gels or each other; and after a given storage time, the construct is gently washed through a size-limiting membrane to separate the platelets from the microgels.

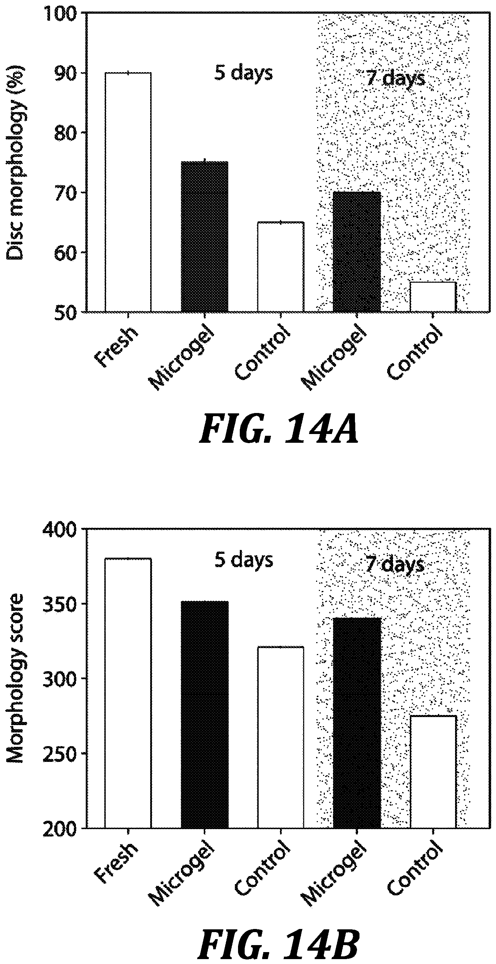

FIGURES 14A-14B compare morphology (platelet morphology score) for fresh platelets, platelets comingled with representative zwitterionic (PCB) microgels of the invention, and platelets under current standard of care conditions. Platelets commingled with representative zwitterionic (PCB) microgels showed an overall higher morphology score after 7 days compared to the current standard of care conditions (control).

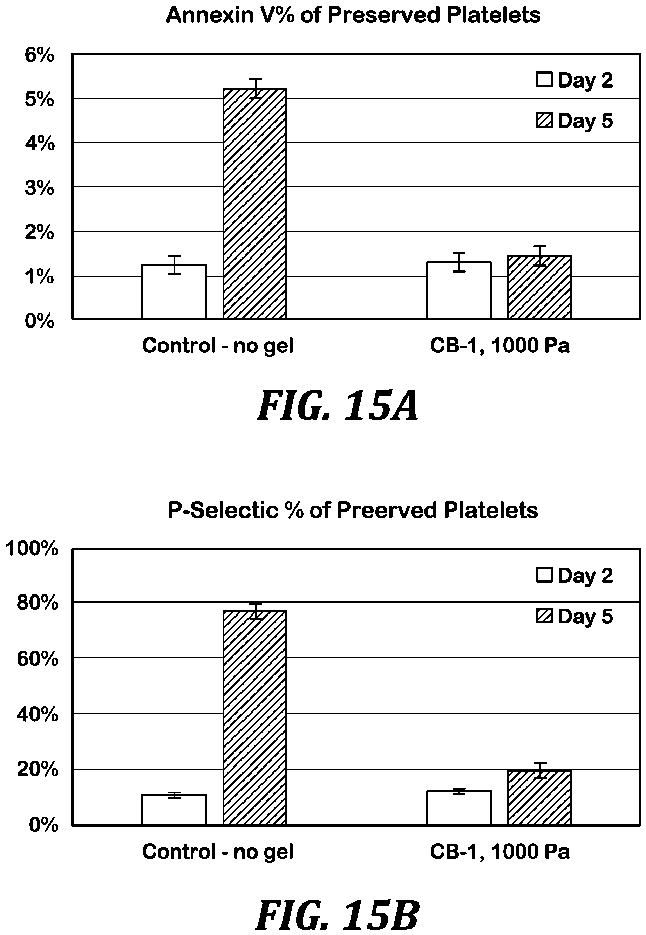

FIGURES 15A-15B compare platelet health after 2 and 4 days of storage comingled with representative zwitterionic (PCB) microgels of the invention, and platelets under current standard of care conditions. Flow cytometry was used to measure annexin (FIGURE 15A) and P-selectin (FIGURE 15B) levels under each condition. Higher levels of these markers after 5 days under current standard-of-care conditions signify reduced platelet health.

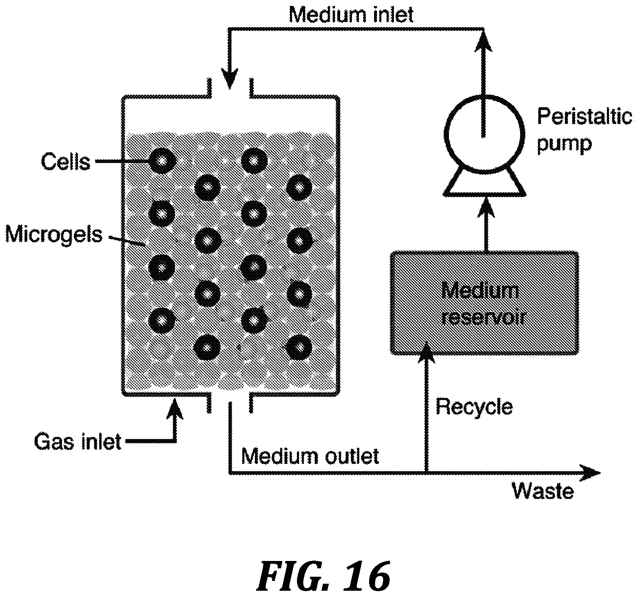

FIGURE 16 is a schematic illustration of a perfusion bioreactor incorporating a microgel support matrix.

### DETAILED DESCRIPTION OF THE INVENTION

Aspects of the disclosure will now be described in more detail. The invention is defined by the claims.

### Cell culture

In another aspect, the disclosure provides a method for cell culturing as defined in the claims. The method comprises culturing a population of cells in a matrix comprising a zwitterionic microgel, as defined in the claims.

In the method, the step of culturing the population of cells can comprise expanding the population. In some embodiments, culturing the population of cells comprises expressing a protein such as an antibody or other biologic. In some embodiments, the cells are selected from pluripotent and multipotent stem and progenitor cells, induced pluripotent stem cells and progenitors or differentiated lineages thereof, hematopoietic cells, genetically engineered cells (vaccines), immune cells and progenitors or differentiated lineages thereof (e.g., T cells, B cells, dendritic cells, antigen-presenting cells), pancreatic islet or other insulin-producing cells, nervous system cells and progenitors, and cardiovascular system cells and progenitors, blood cells (red blood cell, white blood cell, platelets).

In some embodiments, the cells are stem cells or progenitor cells and culturing the population of cells comprises expanding the population without differentiation or change in phenotype. In some embodiments, the cells are stem cells or progenitor cells and culturing the population of cells comprises controlling the differentiation pathway.

### Cell or tissue storage/preservation

In another aspect, the disclosure provides a method for protectively storing a population of cells, a tissue, or an organ, comprising storing a population of cells, a tissue, or an organ as defined in the claims. The method comprises a zwitterionic microgel as defined in the claims to provide stored cells, a stored tissue or a stored organ, wherein the stored cells, stored tissue, or stored organ substantially retains its biological function on storage.

In some embodiments, the cells can be selected from pluripotent and multipotent stem and progenitor cells, hematopoietic cells, genetically engineered cells (vaccines), immune cells and progenitors or differentiated lineages thereof (e.g., T cells, B cells, dendritic cells, antigen-presenting cells), pancreatic islet or other insulin-producing cells, nervous system cells and progenitors, and cardiovascular system cells and progenitors.

In some embodiments, the tissue is muscle (skeletal, smooth, cardiac, vasculature including blood vessels), nerve tissue (peripheral nervous tissue, central nervous tissue including tissue comprised of neuroglia that are astrocytes, microglial cells, ependymal cells, oligodendrocytes, satellite cells, or Schwann cells), connective tissue (cartilage, elastic cartilage, fibrocartilage, bone tissue, white adipose tissue, brown adipose tissue, fascia, blood), subcutaneous tissue, or epithelial tissue (squamous epithelium, cuboidal epithelium, columnar epithelium, stratified epithelium, pseudostratified epithelium, transitional epithelium).

In some embodiments, the organ is kidney, heart, brain (cerebrum, cerebral hemispheres, dencephalon), brainstem (midbrain, pons, medulla oblongata, cerebellum, spinal cord, ventricular system, choroid plexus), esophagus, pharynx, salivary glands (parotid glands, submandibular glands, sublingual glands), stomach, small intestine (duodenum, jejunum, ileum), large intestine, liver, gallbladder, pancreas, nose (nasal cavity, pharynx, larynx, trachea, bronchi, lungs), Ureters, bladder, urethra, arteries, veins, capillaries, lymphatic vessel, lymph node, bone marrow, thymus, spleen, gut-associate lymphoid tissue (tonsils), eye, ear, olfactory epithelium, tongue, or skin.

The cells, tissue, or organ are stored in the absence of a cryoprotectant, or in the presence of an osmolyte.

In some embodiments, the cells are isolated for use by filtration from the microgels.

### Zwitterionic microgel

The zwitterionic microgel encompassed in any of the methods disclosed herein is now described in more detail. In any of the methods described herein, the zwitterionic microgel can comprise a crosslinked zwitterionic polymer having crosslinking range (crosslink sites relative to monomer) from about 0.005% to about 100% (about 0.01% to about 30%, about 0.01% to about 10%). In some embodiments, the zwitterionic microgel comprises a crosslinked zwitterionic polymer having covalent crosslinks, ionic crosslinks, or crosslinks formed by association of a portion of one zwitterionic polymer with another (zwitterionic fusion). In some embodiments, the zwitterionic microgel comprises degradable crosslinks (e.g., hydrolytic, proteolytic, or other stimuli-responsive or physiologically responsive group).

In some embodiments, the zwitterionic microgel comprises a crosslinked zwitterionic polymer selected from a crosslinked polycarboxybetaine, a crosslinked polysulfobetaine, a crosslinked polyphosphobetaine, and a crosslinked polyphosphorylcholine.

The crosslinked zwitterionic polymer is prepared as recited in the claims.

In some embodiments, the zwitterionic microgel consists of a crosslinked zwitterionic polymer.

In some embodiments, the zwitterionic microgel comprises a crosslinked mixed charge copolymer having a diameter from about 1 micron to about 1000 microns.

In some embodiments, the zwitterionic microgel comprises a crosslinked mixed charge copolymer having crosslinking range (crosslink sites relative to monomer) from about 0.01% to about 50% (from about 0.1% to about 30%, from about 0.1% to about 10%, from about 1% to about 5%).

In some embodiments, the zwitterionic microgel comprises a crosslinked mixed charge copolymer having covalent crosslinks, ionic crosslinks, or crosslinks formed by association of a portion of one mixed charge copolymer with another.

In some embodiments, the zwitterionic microgel consists of a crosslinked mixed charge copolymer.

### Mi crogel

The appended claims are directed to methods of the invention, and describe microgels used within the claimed methods.

The microgel composition is prepared from physical processing of a crosslinked zwitterionic hydrogel or a crosslinked mixed charge hydrogel to provide a microgel composition comprising a plurality of crosslinked zwitterionic or a plurality of crosslinked mixed charge microgel units, respectively.

In some embodiments, the microgel units have a diameter from about 1 micron to about 1000 microns.

In some embodiments, the zwitterionic hydrogel is formed from a polymerizable zwitterionic unit. In some embodiments, the mixed charge hydrogel is formed from polymerizable mixed charge units.

The microgel units are formed from a physical processing selected from cutting, chopping, grinding, grading, templating, rubbing, mincing, extruding or crushing the hydrogel defined in the claims to provide the microgel composition.

In some embodiments, the zwitterionic hydrogel is formed from a polymerizable carboxybetaine, a polymerizable sulfobetaine, a polymerizable phosphobetaine, a polymerizable phosphorylcholine, or mixtures thereof.

In some embodiments, the microgel units comprise crosslinks that are covalent bonds, ionic or zwitterionic fusion interactions including intermolecular forces. In some embodiments, the microgel units comprise degradable crosslinks (e.g., hydrolytic, proteolytic, or other stimuli-responsive or physiologically responsive group).

In some embodiments, the microgel composition is sterilized.

In some embodiments, the microgel composition is lyophilized.

In some embodiments, the microgel composition can be reconstituted from a lyophilized state.

In some embodiments, the microgel composition further comprises a therapeutic or cosmetic agent.

Additional description of certain aspects is now provided.

Hydrogels have numerous biomedical applications owing to their similarity with biological tissues and ease of functional and mechanical tuning. However, bulk hydrogels typically lack viscoelastic or shear-dependent material properties; they cannot be injected through a needle, spread on a surface or tissue, molded into new self-supporting shapes, or easily and reversibly assembled into multicomponent constructs. These properties are desirable for many applications, including tissue adhesives, injectable depots to deliver drugs or therapeutic cells, cell growth and preservation scaffolds, wound-healing materials, biologic stabilization, and cosmetic or reconstructive surgery.

The present invention uses in its methods zwitterionic microgels for injection as well as moldable materials, viscoelastic materials, cell growth and preservation scaffolds, bioadhesive materials to produce dynamic assemblies from these micro-scale hydrogels or microgels. Each microgel unit is of a similar size as most cells. The combination of these small discrete microgel units and their interactions when assembled enable a dynamic material with many unique properties, which are described herein.

### Zwitterionic Microgels

As used herein, the term "zwitterionic microgel" refers to a hydrogel having micron dimensions (i.e., having a diameter that is from about 1 and about 1000 microns) that is a crosslinked zwitterionic polymer (e.g., a polycarboxybetaine, polyphosphocholine, polysulfobetaine, polyphosphobetaine) or a mixed charge polymer (e.g., a substantially electronically neutral copolymer having cationic and anionic repeating units). The microgel can be crosslinked via covalent crosslinks, ionic crosslinks, or crosslinks formed by association of a portion of one zwitterionic (or mixed charge) polymer with another (zwitterionic fusion).

Microgels can be produced primarily from zwitterionic monomers, oligomers, crosslinkers or their precursors, such as carboxybetaines, sulfobetaines, phosphobetaines or phosphocholines, or combinations of cationic and anionic monomers (including mixed charge peptides such as those comprising E and K), using various production methods for many different applications; these are described below with carboxybetaine as an example.

The microgel size is important for several reasons: to realize desirable bulk material properties, injection capabilities, and use as a cell growth and/or preservation scaffold material. Important cell types and multicellular structures for growth and preservation vary in size from about 2 µm (platelets) to about 100 µm (pancreatic islets), with the average cell around 20 µm. For optimal cell support without restricting growth and for easy in separation of cells from microgels, microgels should also be near this size range. For injection and flow properties such as through standard-gauge needles, microgels smaller than about 500 µm are required, depending on particle flexibility and other factors. Other bulk material properties such as spreadability also require each discrete microgel to be about 1000 µm or smaller for the aggregate material to have viscoelastic behavior.

This disclosure describes a zwitterionic microgel. These microgels are crosslinked hydrated polymeric structures of approximate length scale **D** (micron dimensions), primarily composed of zwitterionic polymers **(Z)ₙ** (e.g., having zwitterionic or mixed charge repeating units prepared from polymerization of zwitterionic monomers, such as a polymerizable carboxybetaine, a polymerizable sulfobetaine, a polymerizable phosphobetaine or a polymerizable phosphocholine, or mixtures thereof, or the copolymerization of cationic and anionic monomers, respectively). As noted above, any crosslinking mechanism **X,** may be sufficient. One crosslinking strategy is referred to as "zwitterionic fusion" and integrates strong hydration, intermolecular zwitterion pair attraction, and H-bonding between side chains and backbone amides to facilitate time-independent self-healing in some zwitterionic materials, as described in Jiang et al., Biomaterials, 35, 2014, 3926.

A schematic illustration of a zwitterionic microgel is shown below.

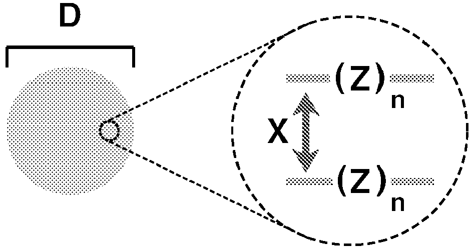

For these microgels, **D,** the average diameter or size of each crosslinked discrete structure, is between about 1 µm (micron) and about 1 mm (millimeter). The discrete structures may be roughly spherical, cubical, tubular or any other three-dimensional shape.

This disclosure describes crosslinked zwitterionic microgels prepared from copolymerization of zwitterionic monomers **(Z)** with the zwitterionic crosslinking agent **(X).** The zwitterionic crosslinking agent can be copolymerized with suitable polymerizable monomers and comonomers to provide crosslinked polymers and crosslinked copolymers.

The crosslinked microgels of the invention are crosslinked polymers having repeating groups and crosslinks derived from the zwitterionic crosslinking agent.

Zwitterionic Monomers. The crosslinked microgels of the disclosure may be crosslinked polymers prepared from copolymerization of the zwitterionic crosslinking agent and suitable polymerizable zwitterionic monomers. The appended claims are directed to methods of the invention, and describe microgel s used within the claimed methods.

A crosslinked polymer (e.g., microgel) as recited in the claims may have repeating units **(Z)** having formula (I): 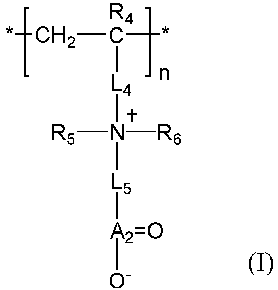 wherein
R₄ is selected from hydrogen, fluorine, trifluoromethyl, C1-C6 alkyl, and C6-C12 aryl groups;
R₅ and R₆ are independently selected from hydrogen, alkyl, and aryl, or taken together with the nitrogen to which they are attached form a cationic center;
L₄ is a linker that covalently couples the cationic center [N⁺(R₅)(R₆)] to the polymer backbone [-(CH₂-CR₄)ₙ-];
L₅ is a linker that covalently couples the anionic center [A₂(=O)O⁻] to cationic center;
A₂ is C, S, SO, P, or PO;
n is an integer from 5 to about 10,000; and
* represents the point at which the repeating unit is covalently linked to either an adjacent repeating unit or the zwitterionic crosslink.

In one embodiment, R₄ is C1-C3 alkyl.

R₅ and R₆ are independently selected from hydrogen, alkyl and aryl, or taken together with the nitrogen to which they are attached form a cationic center. In one embodiment, R₅ and R₆ are C1-C3 alkyl.

In certain embodiments, L₄ is selected from the group consisting of -C(=O)O-(CH₂)ₙ- and -C(=O)NH-(CH₂)ₙ-, wherein n is an integer from 1 to 20. In certain embodiments, L₄ is -C(=O)O-(CH₂)ₙ-, wherein n is 1-6.

In certain embodiments, L₅ is -(CH₂)ₙ-, where n is an integer from 1 to 20.

In certain embodiments, A₂ is C or SO.

In certain embodiments, n is an integer from 5 to about 5,000.

In one embodiment, R₄, R₅, and R₆ are methyl, L₄ is -C(=O)O-(CH₂)₂-, L₅ is -(CH₂)-, A₁ is C, and n is an integer from 10 to about 1,000.

In certain embodiments, **Z** (and polymers **(Z)ₙ**) may be a mixture of polycarboxybetaine-based monomers or polymers and other classes of ionic or non-ionic nonfouling monomers or polymers, or a copolymer of polycarboxybetaine and other classes of ionic or non-ionic monomers, or a mixture or copolymer of cationic and anionic monomers/polymers such that the overall character of the microgel is substantially zwitterionic, mixed charge, or resists protein adhesion and nonspecific interactions (nonfouling).

In addition to the crosslinked polymer (e.g., microgel) having repeating units having formula (1) above, in certain embodiments, the crosslinked polymer as recited in the claims may have zwitterionic crosslinks having formula (II): 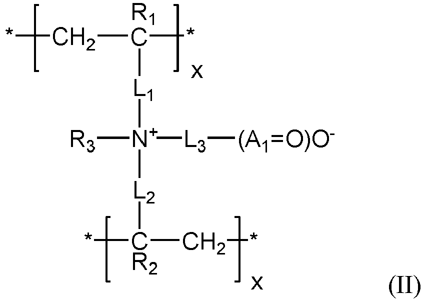 wherein R₁, R₂, R₃, L₁, L₂, L₃, and A₁, are as described above for the zwitterionic crosslinking agent (formula (I)), and x is an integer from about 5 to about 10,000. For the crosslinked hydrogel where R₃ includes a polymerizable group, the hydrogel is further crosslinked through R₃, as shown above (-L₁-CR₁-CH₂- and -L₂-CR₂-CH₂-).

The crosslinked zwitterionic hydrogels of the invention can be prepared by copolymerization of the zwitterionic crosslinking agent with monomers having formula (III):

CH₂=C(R₄)-L₄-N⁺(R₅)(R₆)-L₅-A₂(=O)O⁻ (III)

wherein R₄, R₅, R₆, L₄, L₅, and A₂, are as described above for the repeating unit of formula (II).

Representative crosslinked zwitterionic polymers of the invention have formula (IV):

PB-(L₄-N⁺(R₅)(R₆)-L₅-A₂(=O)O⁻)ₙ (IV)

wherein R₅, R₆, L₄, L₅, A₂, and n are as described above for the repeating unit of formula (I), and PB is the polymer backbone that includes repeating units [formula (I)] and crosslinks [formula (II)].

### Representative Crosslinked Mixed Charge Microgels

Microgels are defined in the claimed methods.

This disclosure describes crosslinked mixed charge copolymers (or microgels) prepared from copolymerization of ion pair comonomers with the zwitterionic crosslinking agent. The appended claims are directed to methods of the invention, and describe microgels used within the claimed methods.

As used herein, the term "mixed charge copolymer" refers to a copolymer having a polymer backbone, a plurality of positively charged repeating units, and a plurality of negatively charged repeating units. In the practice of the invention, these copolymers may be prepared by polymerization of an ion-pair comonomer.

The mixed charge copolymer includes a plurality of positively charged repeating units, and a plurality of negatively charged repeating units. In one embodiment, the mixed charge copolymer is substantially electronically neutral. As used herein, the term "substantially electronically neutral" refers to a copolymer that imparts advantageous nonfouling properties to the copolymer. In one embodiment, a substantially electronically neutral copolymer is a copolymer having a net charge of substantially zero (i.e., a copolymer about the same number of positively charged repeating units and negatively charged repeating units). In one embodiment, the ratio of the number of positively charged repeating units to the number of the negatively charged repeating units is from about 1:1.1 to about 1:0.5. In one embodiment, the ratio of the number of positively charged repeating units to the number of the negatively charged repeating units is from about 1:1.1 to about 1:0.7. In one embodiment, the ratio of the number of positively charged repeating units to the number of the negatively charged repeating units is from about 1:1.1 to about 1:0.9.

Ion Pair Comonomers. In one embodiment, the crosslinked hydrogels of the claimed methods are crosslinked polymers prepared from copolymerization of the zwitterionic crosslinking agent and suitable polymerizable ion pair comonomers.

Representative ion-pair comonomers useful in the invention have formulas (V) and (VI):

CH₂=C(R₇)-L₆-N⁺(R₉)(R₁₀)(R₁₁) X⁻ (V)

CH₂=C(R₈)-L₇-A₃(=O)-O⁻M⁺ (VI)

In this embodiment, the crosslinked polymer (e.g., microgel) has repeating units having formula (VII): 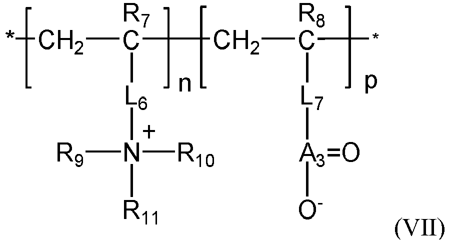 wherein
R₇ and R₈ are independently selected from hydrogen, fluorine, trifluoromethyl, C1-C6 alkyl, and C6-C12 aryl groups;
R₉, R₁₀, and R₁₁ are independently selected from hydrogen, alkyl, and aryl, or taken together with the nitrogen to which they are attached form a cationic center;
A₃(=O)-O⁻) is an anionic center, wherein A₃ is C, S, SO, P, or PO;
L₆ is a linker that covalently couples the cationic center [N⁺(R₉)(R₁₀)(R₁₁)] to the polymer backbone;
L₇ is a linker that covalently couples the anionic center [A(=O)-O⁻] to the polymer backbone;
n is an integer from 5 to about 10,000;
p is an integer from 5 to about 10,000; and
* represents the point at which the repeating units is covalently linked to either and adjacent repeating unit or the zwitterionic crosslink.

In one embodiment, R₇ and R₈ are C1-C3 alkyl.

R₉, R₁₀, and R₁₁ are independently selected from hydrogen, alkyl, and aryl, or taken together with the nitrogen to which they are attached form a cationic center. In one embodiment, R₉, R₁₀, and R₁₁ are C1-C3 alkyl.

In certain embodiments, L₆ is selected from the group consisting of -C(=O)O-(CH₂)ₙ- and -C(=O)NH-(CH₂)ₙ-, wherein n is an integer from 1 to 20. In certain embodiments, L₆ is -C(=O)O-(CH₂)ₙ-, wherein n is 1-6.

In certain embodiments, L₇ is a C1-C20 alkylene chain. Representative L₇ groups include -(CH₂)ₙ-, where n is 1-20 (e.g., 1, 3, or 5)

In certain embodiments, A₃ is C, S, SO, P, or PO.

In certain embodiments, n is an integer from 5 to about 5,000.

In one embodiment, R₇, R₈, R₉, R₁₀, and R₁₁ are methyl, L₆ and L₇ are -C(=O)O-(CH₂)₂-, A₁ is C, and n is an integer from 10 to about 1,000.

In addition to the crosslinked copolymer having repeating units having formula (VII) above, the crosslinked polymer includes zwitterionic crosslinks having formula (II).

Representative crosslinked zwitterionic polymers of the invention have formula (VIII):

PB-[L₆-N⁺(R₉)(R₁₀)(R₁₁)]ₙ[L₇-A₃(=O)-O⁻)]ₚ (VIII)

wherein L₆, N⁺(R₉)(R₁₀)(R₁₁), L₇, A₃(=O)O⁻, n, and p are as described above, and PB is the polymer backbone that includes repeating units [formula (VII)] and crosslinks [formula (II)].

The following is a description of the crosslinking agent, monomers, comonomers, polymers, copolymers, and crosslinks of formulas (I)-(VIII) described above.

In the above formulas, PB is the polymer backbone. Representative polymer backbones include vinyl backbones (e.g., -C(R')(R")-C(R‴)(Rʺʺ)-, where R', R", R‴, and R‴ are independently selected from hydrogen, alkyl, and aryl) derived from vinyl monomers (e.g., acrylate, methacrylate, acrylamide, methacrylamide, styrene). Other suitable backbones include polymer backbones that provide for pendant groups. Other representative polymer backbones include peptide (polypeptide), urethane (polyurethane), and epoxy backbones.

Similarly, in the above formulas, CH₂=C(R)- is the polymerizable group. It will be appreciated that other polymerizable groups, including those noted above, can be used to provide the monomers and polymers of the invention.

In the above formulas, N⁺ is the cationic center. In certain embodiments, the cationic center is a quaternary ammonium (e.g., N bonded to L₄, R₅, R₆, and L₅). In addition to ammonium, other useful cationic centers (R₅ and R₆ taken together with N) include imidazolium, triazaolium, pyridinium, morpholinium, oxazolidinium, pyrazinium, pyridazinium, pyrimidinium, piperazinium, and pyrrolidinium.

R₄, R₅, R₆, R₉, R₁₀, and R₁₁ are independently selected from hydrogen, alkyl, and aryl groups. Representative alkyl groups include C1-C10 straight chain and branched alkyl groups. In certain embodiments, the alkyl group is further substituted with one of more substituents including, for example, an aryl group (e.g., -CH₂C₆H₅, benzyl). Representative aryl groups include C6-C12 aryl groups including, for example, phenyl. For certain embodiments of the above formulas, R₅ and R₆, or R₉, R₁₀, and R₁₁ are taken together with N⁺ form the cationic center.

L₄ (or L₆) is a linker that covalently couples the cationic center to the polymer backbone. In certain embodiments, L₄ includes a functional group (e.g., ester or amide) that couples the remainder of L₄ to the polymer backbone (or polymerizable moiety for the monomers). In addition to the functional group, L₄ can include an C1-C20 alkylene chain. Representative L₄ groups include -C(=O)O-(CH₂)ₙ- and -C(=O)NH-(CH₂)ₙ-, where n is 1-20 (e.g., 3).

L₅ is a linker that covalently couples the cationic center to the anionic group (i.e., (A=O)O⁻). L₅ can be a C1-C20 alkylene chain. Representative L₅ groups include -(CH₂)ₙ-, where n is 1-20 (e.g., 1, 3, or 5).

L₇ is a linker that covalently couples the polymer backbone to the anionic group. L₇ can be a C1-C20 alkylene chain. Representative L₇ groups include -(CH₂)ₙ-, where n is 1-20 (e.g., 1, 3, or 5).

A(=O)-O⁻ is the anionic center. The anionic center can be a carboxylic acid ester (A is C), a sulfinic acid (A is S), a sulfonic acid (A is SO), a phosphinic acid (A is P), or a phosphonic acid (A is PO).

In the above formulas, representative alkyl groups include C1-C30 straight chain and branched alkyl groups. In certain embodiments, the alkyl group is further substituted with one of more substituents including, for example, an aryl group (e.g., -CH₂C₆H₅, benzyl).

Representative aryl groups include C6-C12 aryl groups including, for example, phenyl including substituted phenyl groups (e.g., benzoic acid).

X⁻ is the counter ion associated with the cationic center. The counter ion can be the counter ion that results from the synthesis of the cationic polymers or the monomers (e.g., Cl, Br⁻, I⁻). The counter ion that is initially produced from the synthesis of the cationic center can also be exchanged with other suitable counter ions to provide polymers having controllable hydrolysis properties and other biological properties. Representative hydrophobic counter ions include carboxylates, such as benzoic acid and fatty acid anions (e.g., CH₃(CH₂)ₙCO₂⁻ where n = 1-19); alkyl sulfonates (e.g., CH₃(CH₂)ₙSO₃⁻ where n = 1-19); salicylate; lactate; bis(trifluoromethylsulfonyl)amide anion (N⁻(SO₂CF₃)₂); and derivatives thereof. Other counter ions also can be chosen from chloride, bromide, iodide, sulfate; nitrate; perchlorate (ClO₄); tetrafluoroborate (BF₄); hexafluorophosphate (PF₆); trifluoromethylsulfonate (SO₃CF₃); and derivatives thereof. Other suitable counter ions include hydrophobic counter ions and counter ions having therapeutic activity (e.g., an antimicrobial agent, such as salicylic acid (2-hydroxybenzoic acid), benzoate, lactate.

For the monomers, R₁ and R₂ [formula (I)] and R₄ [formula (III)], is selected from hydrogen, fluoride, trifluoromethyl, and C1-C6 alkyl (e.g., methyl, ethyl, propyl, butyl). In one embodiment, R₁, R₂, and R₄ are hydrogen. In one embodiment, R₁, R₂, and R₄ are methyl.

In certain embodiments, **Z** may be a functionalized carboxybetaine-based monomer, oligomer, or polymer, which in certain embodiments incorporates (a) one of a reactive pair selected from an azide and an alkyne, an azide and an alkene, a thiol and a maleimide, a thiol and an alkene, a thiol and a disulfide, or any other 'click', bioorthogonal, or other reactive pair; (b) a functional group positioned at the terminus of the polymeric structure(s) or along the backbone; and/or (c) a peptide, nucleic acid, protein, antibody, other biomolecule, nanoparticle, microparticle, micelle, liposome, polymersome, drug, drug precursor, or other therapeutic species or drug delivery modality, for surgical applications, cosmetic or aesthetic applications, therapeutic applications, wound-healing applications, drug delivery formulations, cell culture, storage and/or preservation, or regenerative medicine.

### Representative Crosslinked Zwitterionic Microgels

For the microgels, **X** (crosslinking mechanism) is any combination of physical and/or chemical crosslinking mechanisms, within each microgel or between microgels. A schematic illustration of both types of crosslinking (denoted X₁ and X₂) is shown below.

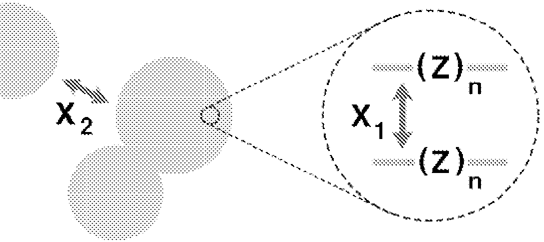

In certain embodiments, **X** (including X₁ and/or X₂ in the schematic above) includes (a) chemical crosslinkers of any structure that are copolymerized with the monomers via a radical-mediated reaction, including commercially available crosslinkers based on polyethylene glycol (PEG), oligoethylene glycol (OEG) or other structures or groups, terminated with two or more acrylate, methacrylate, acrylamide, maleimide or similar reactive groups, or custom synthesized crosslinkers incorporating any functional, reactive, or degradable groups. Optional degradable groups may be selected from disulfide bonds, esters, anhydrides, enzymatically cleavable peptides (such as the matrix metalloproteinase (MMP)-cleavable motifs derived from collagen), or chemistries responsive to external stimuli; (b) bioorthogonal crosslinking chemistries and 'click' chemistries, such as azide/alkyne (including SPAAC) and thiol-ene chemistries, whether through inclusion as functional groups in the main polymer chain(s) or architectures or as separate crosslinking molecules; (c) physical interactions of any type including ionic interactions, hydrogen bonding, hydrophobic interactions, interactions with biomolecules or nanoparticles of a natural or synthetic origin, or any other reversible or nonreversible physical interactions; (d) crosslinks formed by association of a portion of one zwitterionic polymer with another (zwitterionic fusion); or (e) any combination of the above crosslinking mechanisms.

In certain embodiments, **X** is a crosslinking molecule, oligomer, or polymer incorporating one or more zwitterionic or mixed-charge moieties or precursors thereof, or a mixture of these molecules (a) that may be selected from carboxybetaines, sulfobetaines, phosphobetaines, and phosphorylcholines; (b) that may or may not incorporate degradable groups such as disulfide bonds, esters, or stimuli-responsive groups or degradable peptides.

In certain embodiments, the microgels are produced at or near their final size **D** during the polymerization reaction, for example, in a process such as microemulsion polymerization.

In certain embodiments, the microgels are derived from bulk hydrogels and sized to their final dimensions **D** after polymerization using any processing step to grind, extrude, mince, cut, or pellet the bulk hydrogels to discrete units of approximate diameter **D.**

In certain embodiments, the microgels are dried or lyophilized (freeze-dried) to a dehydrated powder for storage, transport, use, or sterilization. In certain of these embodiments, the dried microgel is rehydrated with any aqueous fluid, including but not limited to water, saline or ionic solutions, cell growth or preservation media containing or not containing cells, or any other physiologically relevant solution which may contain drugs, protein therapies, nucleic acid therapies, cells, nanoparticles, or microparticles.

### Zwitterionic Microgel Assemblies

As used herein, the term "zwitterionic microgel assembly" refers to two or more (typically about 100 or more) zwitterionic microgels in contact forming an aggregated gel material.

This disclosure describes a zwitterionic microgel assembly, which is a material formed from two or more microgels assembled through interactions between each discrete microgel resulting in a bulk material. The assembly may have one or more of the following properties: (a) both viscous and elastic properties under different circumstances or other non-Newtonian flow properties; (b) the ability to be spread on surfaces and tissues and reversibly adhere to said objects; (c) the ability to be injected through standard needles typically used in clinical settings; (d) the ability to change viscosity reversibly under differing shear forces; (e) the ability to self-heal upon molding or reconfigurement; and (f) the ability to support other molecules, biomolecules, nanoparticles, microparticles, cells, tissues, or organs as a carrier, scaffold, matrix, storage, or preservation solution or formulation.

The assembly may be a material formed from two or more microgels and includes one or more additional components supported within the assembly. Representative additional components include small molecule drugs, peptides, biomolecules, nanoparticles, microparticles, cells, and tissues.

### Zwitterionic Microgel and Microgel Assembly Formulations and Use

Microgels are as defined in the claimed methods. The microgels and their assemblies, and/or their partially or fully dried or rehydrated compositions, advantageously have many uses. Representative uses include:
providing materials with non-Newtonian behavior (e.g., that exhibits viscoelastic, rheopectic, thixotropic, shear thickening (dilatant), shear thinning (pseudoplastic), and/or Bingham plastic properties);
self-healing materials and/or shape memory materials, or similar classes of 'smart' materials that can repair damage or recover their properties after damage or external stimuli;
antifouling materials or surface coatings to prevent nonspecific protein or other biomolecule adsorption (e.g., for marine applications, drug delivery platforms, biosensors and other medical devices, vascular grafts, intravascular stents, cardiac valves, joint replacements, and other materials and devices that come into contact with physiological environments);
injectable or spreadable materials for biomedical applications;
biocompatible materials used in cell or tissue culture and expansion applications (e.g., as a scaffold, matrix, or other growth substrate in small or large-scale settings and in any container or bioreactor, particularly when cell growth or differentiation must be controlled, expansion without differentiation or phenotype change is desired, or separation of cells and scaffold or matrix material must be done through size-based washing without any additional reagents; and
biocompatible materials used in cell or tissue storage or preservation applications, e.g., as a preservation additive, formulation, scaffold, matrix, surface coating, cryoprotectant, or similar applications.

The microgels and their assemblies can be used as an injectable or spreadable material for biomedical applications, particularly in applications requiring non-Newtonian fluid properties and high biocompatibility, such as (a) injectable or spreadable materials capable of mechanical support, such as those used in cosmetic or reconstructive surgery, blood vessel prostheses, skin repair devices, cochlear replacements, injectable vitreous substances, artificial cartilage, artificial fat, collagen-mimics and other soft tissue-mimics or supports; (b) injectable or spreadable materials with desirable or specific biological interactions with a surface or tissue, particularly when nonspecific interactions should be avoided or a desired balance of nonspecific/specific interactions must be achieved; and (c) injectable or spreadable carriers to deliver and/or protect or shield drugs, biomolecules (e.g., nucleic acids, peptides, proteins, polysaccharides), cells (e.g., pancreatic islets, cardiovascular cells, stem cells, immune cells, blood cells), nanoparticles or microparticles (e.g., PLGA/drug formulations), micelles, liposomes, polymersomes, or other therapeutic species or drug delivery modalities, for surgical applications, therapeutic applications, wound healing, and drug delivery formulations;

The microgels and their assemblies can be used as a scaffold, matrix, or other substrate for the growth, maintenance or expansion of cells, tissues, or organs in which the microgel constructs can be grown using any culture or maintenance method or apparatus including any type of bioreactor, and can be derived from lineages including, but not limited to:
(a) pluripotent and multipotent stem and progenitor cells, including (1) embryonic stem cells (ESCs), tissue-derived stem cells (e.g., from skin, blood, or eye), hematopoietic stem and progenitor cells (HSPCs) derived or purified from umbilical cord blood or bone marrow, mesenchymal stem cells, or induced pluripotent stem cells (iPSCs), (2) genetically modified or transfected stem and progenitor cells; and (3) cancer stem cells (CSCs);
(b) hematopoietic cells typically circulating in human blood, including red blood cells (erythrocytes), white blood cells (leukocytes) and platelets (thrombocytes);
(c) immune cells and progenitors or differentiated lineages thereof, including (1) T cells expressing the CD8 surface glycoprotein, particularly including naive cytotoxic T lymphocytes (CTLs or T_{C}s) and differentiated or activated lineages thereof including central memory (T_{CM}) T cells; (2) T cells expressing the CD4 surface glycoprotein, particularly including naive helper T lymphocytes (T_{H}0), and differentiated or activated lineages thereof including T_{H}1, T_{H}2, T_{H}9, T_{H}17, T_{FH}, T_{REG}, and central memory (T_{CM}) T cells; (3) regulatory T cells (T_{REG}) from any source, either natural Tregs or induced Tregs; (4) natural killer T cells (NKT cells); (5) chimeric antigen receptor T cells (CAR-T); and (6) genetically modified T cells; (6) B cells; (7) dendritic cells, and (8) other antigen-presenting cells (APCs) or immune cells not specifically listed above;
(e) pancreatic islet or other insulin-producing cells and β-cells useful in the treatment and management of diabetes;
(f) nervous system cells and progenitors;
(g) cardiovascular system cells and progenitors; and
(h) other cells, particularly those useful in the fields of immunotherapy, regenerative medicine, hematologic diseases or malignancies, or cancer vaccines or treatments.
(i) tissues, including muscle (skeletal, smooth, cardiac, vasculature including blood vessels), nerve tissue (peripheral nervous tissue, central nervous tissue including tissue comprised of neuroglia that are astrocytes, microglial cells, ependymal cells, oligodendrocytes, satellite cells, or Schwann cells), connective tissue (cartilage, elastic cartilage, fibrocartilage, bone tissue, white adipose tissue, brown adipose tissue, fascia, blood), subcutaneous tissue, or epithelial tissue (squamous epithelium, cuboidal epithelium, columnar epithelium, stratified epithelium, pseudostratified epithelium, transitional epithelium)
(j) organs, including kidney, heart, brain (cerebrum, cerebral hemispheres, dencephalon), brainstem (midbrain, pons, medulla oblongata, cerebellum, spinal cord, ventricular system, choroid plexus), esophagus, pharynx, salivary glands (parotid glands, submandibular glands, sublingual glands), stomach, small intestine (duodenum, jejunum, ileum), large intestine, liver, gallbladder, pancreas, nose (nasal cavity, pharynx, larynx, trachea, bronchi, lungs), Ureters, bladder, urethra, arteries, veins, capillaries, lymphatic vessel, lymph node, bone marrow, thymus, spleen, gut-associate lymphoid tissue (tonsils), eye, ear, olfactory epithelium, tongue, or skin.

The microgels and their assemblies can be used as a biocompatible material, scaffold, formulation component or contacting material for any method of preserving cells or tissues or retaining their biological function for clinical or military utility, particularly for cell types that are difficult to preserve with conventional methods such as blood cells (e.g., platelets and red blood cells) for extended time periods, at room or low temperatures, in whole blood or preservation solutions, and with or without the presence of DMSO, glycerol, glycine betaine or other osmolytes or cryoprotectants.

The preparation, characterization, and representative uses of the zwitterionic microgels and zwitterionic microgel assemblies are described in Examples 1-3.

The following is a description of embodiments of the invention which is defined by the claims.

Design and production of ZIP hydrogels. To create zwitterionic microgels in bulk (FIGURE 1A-1D), we first produced macroscopic PCB hydrogels using a photopolymerization casting method similar to one previously reported (Jiang et al., Biomaterials, 32, 2011, 6893). All hydrogels in this work were constructed from pure polycarboxybetaine acrylamide (PCB-1 or PCB-2) with various carboxybetaine acrylamide crosslinker (CB-X) concentrations (FIGURE 2B). Similar PCB hydrogels, containing pure zwitterionic monomers and crosslinkers, have been previously reported to evade the foreign body reaction upon subcutaneous implantation in Jiang et al., Nature Biotechnology, 31, 2013, 553. In addition, while PCB cell scaffolds have been shown to preserve stem cell multipotency, this relies on all components being zwitterionic: adding a hydrophobic crosslinker can trigger cell differentiation (Jiang et al., Angewandte Chemie International Edition, 53, 2014, 12729). Therefore, we aimed to use exclusively PCB-based components when designing injectable and malleable zwitterionic hydrogels. Extensive material modifications would add synthetic complexity, batch variability, and hydrophobicity, which could compromise the desirable properties. The equilibrium water content (EWC) of all bulk PCB hydrogels after several days of equilibration was 97-99.5 wt%. After complete equilibration, we processed the bulk hydrogel sheets into microgels by repeatedly extruding them through micronic steel mesh using a custom-fabricated piston assembly. Changing the mesh pore size allowed us to easily tune the size of equilibrium-swollen microgels; in this embodiment, microgels were processed to have a mean diameter slightly greater than most human cells (15 - 30 µm), though the same strategy can be used to target any micro-scale size. The overall design and production process is straightforward and amenable to small or large scales. We observed assemblies of these microgels to form malleable but self-supporting constructs with consistent properties between batches. We refer to this class of reconstructed dynamic gels as *"ZIP"* (Zwitterionic Injectable Pellet) hydrogels or constructs. Their self-healing behavior is due to the zwitterionic fusion mechanism previously reported (FIGURE 2D). Zwitterionic fusion is unique because it is both time- and pH-independent; in single-charged or non-ionic self-healing materials, hydrophobic surface reconstruction or a pH-dependent charge barrier limits healing. Even with these key advantages, zwitterionic fusion encounters a geometric limitation; only polymer chains near the exposed surface of crosslinked PCB hydrogels have sufficient mobility to rearrange and form the new supramolecular interactions that lead to bulk healing (e.g. between two large cut segments). In ZIP constructs, each internally crosslinked microgel participates in dynamic healing interactions with many neighboring pellets in 3-D. At this scale, each individual gel is large enough to retain strength and elasticity from its covalent crosslink network, yet small enough to involve a higher percentage of its polymer chains in dynamic interactions. While the molecular identity of each microgel is the same as the bulk hydrogel, inter-microgel zwitterionic fusion interactions are key to the tunable rheological behavior of the aggregate material. All ZIP hydrogels in this work were injectable through a 25-G needle and rapidly reverted to a self-supporting gel state in an inverted vial or on a flat surface. Images showing examples of these key characteristics are shown in FIGURE 3. The schematics in FIGURE 4 give an overview of some of the promising clinical applications of ZIP-based formulations.

Rheological behavior of ZIP hydrogels. To quantitatively characterize the injectability and self-healing capabilities of ZIP hydrogels, we examined their viscoelastic characteristics using rheology. First, we conducted dynamic oscillatory frequency sweeps on ZIP gels based on PCB-1 and PCB-2, each incorporating 0.1 mol% of CB-X crosslinker. This data showed the storage modulus (G', used as a measure of strength) to be dominant over the loss modulus (G") over the full frequency range examined (0.1 - 100 rad s⁻¹), suggesting that both reconstructed ZIP materials behaved like an elastic hydrogel (FIGURE 5). Notably, PCB-2 ZIP gels displayed higher G' values at each crosslinking level compared with PCB-1, especially at very low crosslinker content (0.025 mol% CB-X). In addition, PCB-2 constructs all exhibited lower tan δ values (around 0.25) compared with PCB-1 (around 0.6). As tan δ is inversely associated with elasticity, this indicated PCB-2 produced more elastic constructs-these results were consistent across all crosslinking concentrations and between differently-crosslinked PCB-1 and PCB-2 samples with similar G', suggesting zwitterionic fusion interactions endow PCB-2 constructs with additional bulk strength and elasticity. We further characterized ZIP gels with oscillatory strain sweep and step-strain experiments. FIGURES 6A-6D show shear-thinning rheological properties of representative microgel compositions of the invention as measured by oscillatory strain sweep tests: PCB-1 based zwitterionic microgels, PCB-2 based zwitterionic microgels, MPC based zwitterionic microgels, and mixed charge microgels. In all subfigures, storage (G', solid markers) and loss (G", open markers) moduli are plotted as strain increases from <1% to 100%. We found G' to dominate over G" in these and other representative formulations at low strains (0.1 - 1%). The complex viscosity and G' began to decrease as we pushed the strain towards 100%, with most samples exhibiting a crossover point (tan δ = 1) between 10% and 30% strain. Above this strain, G" becomes dominant and the gels begin to adopt liquid-like behavior as inter-microgel associations dynamically break and re-form. FIGURES 7A-7D compare self-healing properties of representative microgel formulations as measured by rheological step-strain tests. In all subfigures, storage (G', solid markers) and loss (G", open markers) moduli are plotted as the strain is toggled between 1% (white background) and 300% (shaded background). Representative formulations show inversion of G' and G" at high strain followed by a rapid recovery of elastic properties when low strain is returned. This is indicative of efficient self-healing across all samples.

Sterilization and freeze drying of microgels. Freeze-drying ZIP gels to store them as sterile lyophilized powders is practical and enables dramatically simplified formulation of many drug- or cell-encapsulating composite constructs In this embodiment, hydrogels were sterilized post-equilibration and processing by immersion in >70% EtOH, which had no impact on their appearance or behavior when reconstituted with sterile water for lyophilization. Autoclaving, ethylene oxide gas, and gamma irradiation are also suitable methods to sterilize zwitterionic hydrogels, as described in Jiang et al., Biointerfaces, 12, 2017, 02C411. While other types of macroscopic hydrogels have been lyophilized to create porous "top-down" scaffolds for drug delivery or cell attachment, the freeze-drying process is known to irreversibly change some aspects of their structure and behavior. Lyophilized gels commonly require immersion in water for hours to days to fully rehydrate, and even then, fail to reach their original water content and display uneven shapes, surface roughness, and modified material properties. Due to the particularly strong hydration of zwitterionic materials, we believed lyophilization would not have a detrimental impact on ZIP hydrogels. We tested this by freeze-drying each ZIP formulation to desiccated powder. As the EWC of lightly-crosslinked PCB hydrogels is near 99%, each milliliter of gel only contains around 10 mg of dry material, which could be combined from multiple batches for simplified storage and formulation. Notably, when we mixed dry ZIP powder with the volume of pure water necessary to return the gels to their original EWC, hydration completed within seconds. The rapidly rehydrated gel constructs retained their transparency, homogeneity, and practical attributes (e.g., injectability and self-healing). Thus, we conducted further rheological testing to compare samples before and after lyophilization and rehydration. As highlighted in FIGURE 10A, freeze-drying PCB-1 and PCB-2-based ZIP constructs had no effect on their bulk strength (G') or elasticity (tan δ) post-rehydration. In addition, their high self-healing efficiency was unchanged, with no difference in step-strain recovery time after multiple cycles (FIGURE 10B).

Injectable drug depot formulations (comparative example) that release therapies at a predictable rate over a designated period of time represent one example of a clinical need motivating the development of biocompatible injectable hydrogels. Reconstitution of drug-loaded PLGA or PCL microspheres in an injectable crosslinked gel facilitates accurate volumetric dosing and keeps the formulation localized at the injection site. Based on the ability of zwitterionic hydrogel to mitigate the foreign body reaction, it was envisioned that they may also be helpful in shielding otherwise-immunogenic materials from immune recognition and attack in a composite formulation. To explore one likely application, PLGA microspheres (MS) containing chemotherapeutic drug doxorubicin (DOX) were prepared using a double (W/O/W) emulsion method, tuning the process parameters to achieve smooth particles ~30 µm in diameter (FIGURE 8A) and targeting a 2-3-week controlled release period. DOX-PLGA MS was mixed with ZIP powder and reconstituted this formulation to 40 mg DOX-PLGA MS (containing 2 mg total DOX) per mL of ZIP gel. The lyophilized mix reconstituted to a homogeneous formulation in seconds, with only brief vortexing required during hydration to disperse particles evenly. PLGA MS were held in place by the gel in an inverted vial; no obvious changes, settling, or separation were observed in undisturbed vials for -at least 4 weeks. DOX release rate at 37°C *in vitro* was evaluated by dispensing the ZIP-DOX-PLGA depot (or DOX-PLGA without gel) into porous Transwell inserts suspended in PBS (FIGURE 8B). This was designed to simulate the *in vivo* environment of a subcutaneously injected cancer treatment, as might be administered to prevent tumor recurrence after surgery. The resulting release data are shown in FIGURE 8C. Both ZIP-formulated and control MS samples released about 90% of their total DOX cargo within two weeks, with an insignificant difference in overall release. This indicates the injectable gel did not significantly inhibit or accelerate PLGA hydrolysis and erosion overall, and that DOX could diffuse out through the zwitterionic matrix. It is worth noting that the ZIP-DOX-PLGA formulation seemed to show a lower level of 'burst' release in the first 24 h, which would be another advantage of ZIP depot formulations if found to extend to *in vivo* studies.

In recent years, biologic protein drugs such as therapeutic enzymes and monoclonal antibodies have grown to dominate the pharmaceutical landscape. These drugs can precisely target many debilitating diseases, but remain expensive and are plagued by short circulation half-lives and immunogenic issues. Conjugating zwitterionic PCB to proteins or encapsulating them in individual PCB nanogels has been demonstrated to improve their stability, maintain their bioactivity, and mitigate immunogenic reactions *in vivo.* The zwitterionic moiety in PCB, glycine betaine, is widely known to stabilize protein structures and prevent denaturation and aggregation. Reconstituting ZIP powder with an enzyme solution provides an 'active enzyme gel' for localized injectable or topical biologic therapies. This concept is illustrated in FIGURE 9A. As pictured IN FIGURE 9C, when the model enzyme β-Lactamase (B-La) was mixed with ZIP gel and injected into a small amount of colorimetric substrate nitrocefin, the enzyme catalyzed substrate conversion as intended inside the healed gel construct. This was followed by quantitatively comparing the maximum activity of B-La (Vₘₐₓ) inside a ZIP gel and in buffer. No difference was observed in the substrate conversion rates, showing this simple formulation strategy may be useful for topical biologic delivery without harming activity (FIGURE 9B).

For comparison, an injectable hydrogel (comparative example) based on temperature-responsive Pluronic PEG-PPG-PEG triblocks was formulated with B-La. This alternative formula reduced the enzyme activity by over half, consistent with the well-known and deleterious effects of high PEG content on protein bioactivity.

3D cell culture and protection in injectable ZIP scaffolds. To evaluate the suitability of ZIP constructs for 3D cell culture and preservation, we reconstituted sterile ZIP powder (PCB-1 based, CB-X=0.05%) with growth media containing naive CD4⁺ T helper (Tₕ) cells. This rapidly produced a malleable soft gel scaffold with the Tₕ cells suspended in the rehydrated ZIP matrix. We used porous well plate inserts (8 µm pore size) to support the cell-hydrogel constructs while keeping them equilibrated with the surrounding medium; this model allowed the medium and biochemical factors to be refreshed without disturbing the cell population. At one- and two-week time points, we transferred the constructs to 40 µm cell filters and gently flushed excess buffer through to separate the cells from the gel and allow viability and functional analysis of the Tₕ populations. In this example, we did not strive for rapid expansion of the cell populations (which grew 2-4 fold), but focused on preserving cell functionality, which is paramount during *in vitro* culture of cells grown for cell-based therapies. While viabilities of the overall populations were similar to control cultures grown in flasks after both time points (FIGURE 11A), a significantly higher percentage of cells continued to express CD45RA (naive T-cell marker) after both one and two weeks of ZIP culture (-70%) compared to control flasks (-25%) (FIGURE 11B). This general concept-the rapid creation of malleable, cell-preserving constructs-could be adapted to many scales and applications, and is far simpler than the *in situ* encapsulation chemistries most common in tissue engineering research. In particular, its simplicity would translate well to a clinical or biomanufacturing setting, as no specialized chemistry knowledge or conditions are required. The ability of ZIP culture to maintain naive markers is reminiscent of previous reports describing how zwitterionic hydrogels are capable of restraining stem cell differentiation. However, the simplicity of the ZIP platform makes it more suitable for clinical translation, and these reconstituted scaffolds are a promising strategy to culture and preserve many human cell lines while maintaining their therapeutic potency.

As ZIP constructs proved to be a capable and cytocompatible platform for 3-D cell culture, the ability to inject these constructs directly could prove useful for all-in-one formulation of regenerative therapies. Additionally, several reports by Heilshorn et al have highlighted the protective role some injectable hydrogels can play in shielding therapeutic cells from shear damage as they pass through a needle (Heilshorn et al., Tissue Engineering Part A, 18, 2012, 806.). In general, soft shear-thinning hydrogels (G' ~ 100 Pa) have been reported to give the best protection. Healthy HEK 293T cells were resuspended in PBS at 10⁶ cells mL⁻¹ and used to reconstitute an appropriate amount of ZIP powder (PCB-1 based, CB-X=0.05%) to a gel while gently mixing. Along with being the same ZIP formulation used for Tₕ cell culture experiments, it was also selected to match the rheological attributes of other cell-protective hydrogels. The ZIP-cell construct and a control suspension in PBS were carefully transferred to a 1 mL syringe and injected into a new well plate through a 28G needle. Promisingly, no significant decrease in viability was seen in the ZIP-protected formulation, while a 25-30% decrease was observed in the control (buffer-only) sample. Fluorescent micrographs of stained cells and quantified viability data are shown in FIGURES 12A - 12B. As the same gel formulation supported cell expansion with minimal functionality loss, and protected cells during needle flow, it follows that ZIP constructs could serve as an all-in-one solution for common problems in the practical implementation of cell-based therapies.

In summary, this disclosure describes a simple and versatile strategy to create shear-thinning and self-healing "zwitterionic injectable pellet" (ZIP) hydrogels based on reconstructed microgel assemblies and zwitterionic fusion. Importantly, these gels consist purely of carboxybetaine polymers and crosslinker, are straightforward to make at any scale, and can be simply sterilized and lyophilized for long-term storage and facile reconstitution. Injectable and malleable ZIP formulations can easily be created for many clinical applications, containing therapeutic cells, drug-loaded microspheres, or biologics. As they show promise for injectable filler materials, drug delivery, and even 3-D T-cell culture and preservation, ZIP hydrogels present a versatile platform for a wide variety of clinical applications requiring biocompatible injectable materials.

As used herein, the term "about" refers to ±5% of the specified value.

The following examples are provided for the purpose of illustrating the invention.

### EXAMPLES

### Example 1

### Preparation, Characterization, and Use of Representative Zwitterionic Microgels

In this example, methods for preparing, characterizing, and using representative zwitterionic microgels of the invention are described.

Microgel production. To prepare zwitterionic microgel constructs, bulk zwitterionic hydrogels were prepared using a photopolymerization method. Carboxybetaine acrylamide (CBAA) monomer (2.5 M), CBAA-X crosslinker (0.01-1% mol/mol), and photoinitiator 2-hydroxy-1-[4-(2-hydroxyethoxy)phenyl]-2-methyl-1-propanone (I2959) were dissolved in water, mixed well, and degassed under vacuum. The concentrated solution was then cast into a 1-mm thick glass mold and polymerized in a Spectroline XL-1500 UV oven. The resulting hydrogels were equilibrated in water for several days to remove any unreacted reagents and allow swelling. A library of these parent hydrogels was generated using either CBAA-1 or CBAA-2 monomer and several CBAA-X crosslinker concentrations between 0.025 and 1 mol%, relative to CBAA monomer. Bulk hydrogels were then converted into zwitterionic microgels; equilibrated bulk gels were cut into pieces and placed into an extrusion apparatus consisting of a tightly-fit piston and cylinder capped with a section of micronic steel Dutch-weave mesh, and progressively extruded through meshes of decreasing pore size from 120 µm to 25 µm. At the final mesh size used, the material was extruded at least three times to improve pellet size homogeneity. The final pellets were sterilized with progressive ethanol precipitation, re-swollen with sterile water, and lyophilized for long-term storage.

Rheology. Dynamic viscoelasticity properties of all ZIP constructs were measured with an Anton Paar Physica MCR 301 Rheometer using parallel plates of 40-mm diameter and a plate-to-plate distance of 900 µm. For each experiment, G', G", complex viscosity (η) and tan δ (G"/G') were recorded. Dynamic oscillatory frequency sweeps were conducted at a constant 10% strain over a frequency range of 0.1-100 rad/s at 25°C. Oscillatory strain sweeps were conducted at a constant 1 rad/s frequency over a 0.1% to 100% strain at 25°C. Step-strain experiments were conducted by toggling the strain between 1% and 300% for three or more cycles.

Lyophilization and formulations. ZIP gels were lyophilized to powder and further analyzed for their rheological behavior after reconstitution. To make doxorubicin (DOX)-loaded microgel constructs, DOX (Sigma-Aldrich) was encapsulated in PLGA using a W/O/W double emulsion method adapted from several similar protocols (Merkle et al., Iranian Journal of Pharmaceutical Research, 10, 2011, 203; McCall and Sirianni, Journal of Visualized Experiments, 2013, 51015.), with total drug loading quantified by dissolving particles in DMSO and measuring DOX absorbance (λ=480 nm) with a BioTek Cytation 5 microplate reader, while microsphere surface characterization was done via SEM. DOX-PLGA microspheres were mixed with ZIP powder to a reconstituted formulation consisting of 40 mg DOX-PLGA (containing 2 mg total DOX) per mL of ZIP gel. Drug release rates were evaluated at 37°C *in vitro* by dispensing the *ZIP*-DOX-PLGA depot (or DOX-PLGA microspheres without gel) into porous inserts (Corning Transwell, 8 µm pore size) suspended in PBS. The buffer was sampled and replaced at selected time intervals and the cumulative amount of released DOX assayed spectroscopically at 480 nm. Enzyme formulations were produced containing TEM-1 β-Lactamase in ZIP gels, with nitrocefin (Life Technologies) used as a model substrate. Kinetic parameters of ZIP-enzyme formulations were evaluated in UV-transparent 96-well microplates (Corning) at saturating substrate concentration, and enzyme activity (V) was measured using a microplate reader as the initial linear rate of increase in substrate absorbance at 490 nm.

CD4⁺ T-cell culture. Media containing CD4⁺ human T lymphocytes (Lonza) (1 mL, 10⁶ cells/mL) was used to rehydrate 50 mg of lyophilized ZIP powder, and cells were gently mixed well with the gel during hydration. This construct was transferred to a porous tissue culture insert (Corning Transwell, 8 µm pore size) which was then placed in a 12-well plate with cell media even with the level of the ZIP-cells construct. Cells were suspended in RMPI media containing 10% FBS, 1% penicillin and streptomycin. Cells were stimulated to proliferate with Dynabeads Human T-Activator CD3/CD28, at a ratio of 1:1 bead:cell (seeded at 1×10⁶ cells mL⁻¹) at day 1 and day 8, and 30 U mL⁻¹ Interleukin-2 and incubated at 37°C and 5% CO₂ for 7 days. They were then analyzed via fluorescence flow cytometry on a BD LSR II instrument equipped with a 488-nm excitation source and a 530/30-nm band pass filter; CD45RA⁺ cells were labeled with a FITC marker having excitation and emission peaks of 525 nm. Proliferation analyses were performed using FlowJo software for isotype IgG1 controls tagged with FITC and cell samples within the ZIP construct. Cells stained with CD45RA were visualized by fluorescence intensity peaks to evaluate lineage and phenotype of T cells after 7 days and 14 days and compared to controls.

Cell injection. To evaluate cell protection during needle flow, healthy HEK 293T cells were resuspended in PBS at 10⁶ cells mL⁻¹ and used to rehydrate an appropriate amount of ZIP powder (PCB-1, CB-X = 0.05%) to a gel while gently mixing. Then, the soft ZIP-cell construct was carefully transferred to a 1 mL syringe and injected into a well plate through a 28-G needle. A control suspension was left in PBS and also injected. Cells were LIVE/DEAD stained with calcein-AM and ethidium bromide homodimer and imaged with a fluorescent inverted microscope (Nikon T2000U) to assay the viability post-injection.

### Example 2

### Platelet Preservation with Representative Zwitterionic Microgels

In this example, platelet preservation using representative zwitterionic microgels of the invention is described.

Platelets are blood cells that play a key role in clotting and have many other functions. Platelet transfusion is necessary in trauma and blood disorders. Unfortunately, platelets activate and rapidly become therapeutically useless when removed from the bloodstream of a donor and put into storage. The current state-of-the-art protocol calls for room temperature storage under constant gentle agitation, to prevent aggregation and allow even oxygen diffusion. Low temperature refrigerated storage (4°C) paradoxically causes platelets to lose their clotting ability even faster, but the maximum room-temperature storage time is only between 5-7 days. While platelet additive solutions and gas-permeable bags have increased this maximum storage time, nonspecific aggregation, bacterial contamination, and platelets' interactions with each other and the synthetic bag materials still triggers activation and limits storage time.

The present invention provides a "comingled microgel" storage method in which platelets are mixed with representative zwitterionic microgels of the invention (i.e., PCB microgels) to limit aggregation and nonspecific interactions and improve maximum preservation time. An overall schematic showing this method is shown in FIGURE 13.

In the method, fresh platelets in plasma were added to lyophilized microgels in a platelet storage bag, with platelets suspended and supported by the PCB microgels, but not interacting with the gels or each other. After a given storage time, the construct is gently washed through a size-limiting membrane to separate the platelets (about 4 µm diameter) from microgels (about 20 µm diameter). The separated platelets are then analyzed for marker expression, clotting ability, and morphology score.

The improvement seen in platelet morphology score after 7 days of microgel storage vs. the current standard of care condition (control) is shown in FIGURE 14.

Comingled storage with PCB microgels resulted in an overall higher morphology score after 5 and 7 days compared to the current state-of-the-art condition (control). The morphology score quantifies the percentage of platelets retaining a discoid form and is used as a simple indicator of platelet health. This score, which can reach a maximum value of 400, is equal to 4*(disc%) + 2*(spheres%) + (dendrite%).

Platelet health can also be analyzed using flow cytometry analysis of two markers: Annexin V and P-selectin. Annexin V is a cellular protein used as an indicator of cellular apoptosis; the mechanism involves the ability of Annexin V ability to bind to phosphatidylserine. P-Selectin, a cell adhesion molecule, or CAM, is detected on the surface of activated endothelial cells or platelets, which further indicates the number of usable platelets. The improvement seen in these markers after 5 days of comingled microgel storage is shown in FIGURE 15. Comingled PCB-1 microgels do not increase apoptosis in stored platelets and annexin is significantly lower at day 5 when compared to the current standard of care condition (control). P-selectin levels are also significantly lower at 5 days when compared to the control, indicating more platelets are unactivated, and therefore still usable for donation.

### Example 3

### Bioreactor

In this example, biomanufacturing or industrial-scale cell culture or expansion using representative zwitterionic microgels of the invention is described.

Currently, there has been limited success in expanding stem cell populations and other cells relevant to immunotherapy such as T cells in conventional bioreactors while maintaining their multipotency and/or therapeutic activity. Most materials present in reactors, including optimized biomaterials and modified surfaces, provide nonspecific interactions with cells that trigger phenotype change, contribute to cellular senescence, or require damaging encapsulation reactions and recovery procedures. Shear damage in stirred-tank reactors also limits growth. Pure zwitterionic hydrogels can maintain stem cell multipotency for an unprecedented length of time, and support their expansion at a small scale while protecting them from shear damage. To support cell expansion for a long period of time in continuous culture, zwitterionic microgels can be used as a growth matrix in a perfusion bioreactor. FIGURE 16 illustrates a representative type of bioreactor matrix. A pumping system is connected to a cell culture bag or porous vessel, and a cell-microgel slurry is injected into the reactor and maintained on an orbital shaker at 37°C and 5% CO₂. The pump delivers fresh media at 30 min intervals at a flow rate of 30 mL/min, for semi-continuous delivery. Cultured cells are harvested via size-dependent filters and then analyzed for phenotype and cell function after different time points.

## Claims

1. A method for protectively storing a population of cells, a tissue, or an organ, comprising storing a population of cells, a tissue, or an organ in a zwitterionic microgel to provide stored cells, a stored tissue or a stored organ, wherein the stored cells, stored tissue, or stored organ substantially retain their biological function on storage;
wherein the zwitterionic microgel comprises a microgel composition prepared from physical processing of a crosslinked zwitterionic hydrogel or a crosslinked mixed charge hydrogel to provide a microgel composition comprising a plurality of crosslinked zwitterionic or a plurality of crosslinked mixed charge microgel units; and
wherein the physical processing comprises cutting, chopping, grinding, grading, templating, rubbing, mincing, extruding, or crushing the crosslinked zwitterionic hydrogel or the crosslinked mixed charge hydrogel to provide the microgel composition.

2. The method of Claim 1, wherein the cells are selected from pluripotent and multipotent stem and progenitor cells; hematopoietic cells; genetically engineered cells, such as vaccines, immune cells and progenitors or differentiated lineages thereof such as T cells, B cells, dendritic cells, antigen-presenting cells; pancreatic islet or other insulin-producing cells; nervous system cells and progenitors; and cardiovascular system cells and progenitors.

3. The method of Claim 1 or Claim 2, wherein the tissue is muscle, such as skeletal, smooth, cardiac, vasculature including blood vessels; nerve tissue, such as peripheral nervous tissue, central nervous tissue including tissue comprised of neuroglia that are astrocytes, microglial cells, ependymal cells, oligodendrocytes, satellite cells, or Schwann cells; connective tissue, such as cartilage, elastic cartilage, fibrocartilage, bone tissue, white adipose tissue, brown adipose tissue, fascia, blood; subcutaneous tissue; or epithelial tissue, such as squamous epithelium, cuboidal epithelium, columnar epithelium, stratified epithelium, pseudostratified epithelium, transitional epithelium.

4. The method of any one of the preceding Claims, wherein the organ is kidney; heart; brain including cerebrum, cerebral hemispheres, dencephalon; brainstem including midbrain, pons, medulla oblongata, cerebellum, spinal cord, ventricular system, choroid plexus; esophagus; pharynx; salivary glands, such as parotid glands, submandibular glands, sublingual glands; stomach; small intestine including duodenum, jejunum, ileum; large intestine; liver; gallbladder; pancreas; nose including nasal cavity, pharynx, larynx, trachea, bronchi, lungs; ureters; bladder; urethra; arteries; veins; capillaries; lymphatic vessel; lymph node; bone marrow; thymus; spleen; gut-associate lymphoid tissue, such as tonsils; eye; ear; olfactory epithelium; tongue; or skin.

5. The method of any one of the preceding Claims, wherein the cells, tissue, or organ are stored in the absence of a cryoprotectant, or in the presence of an osmolyte.

6. The method of any one of the preceding Claims, wherein the cells are isolated for use by filtration from the microgel.

7. The method of any one of the preceding Claims, wherein the zwitterionic microgel comprises a crosslinked zwitterionic polymer having crosslinking range from about 0.005% to about 100%, such as about 0.01% to about 30%, or about 0.01% to about 10%.

8. The method of any one of the preceding Claims, wherein the zwitterionic microgel comprises a crosslinked zwitterionic polymer having covalent crosslinks, ionic crosslinks, or crosslinks formed by association of a portion of one zwitterionic polymer with another.

9. The method of any one of the preceding Claims, wherein the zwitterionic microgel comprises degradable crosslinks, such as hydrolytic, proteolytic, or other stimuli-responsive or physiologically responsive group.

10. The method of any one of the preceding Claims, wherein the zwitterionic microgel comprises a crosslinked zwitterionic polymer selected from a crosslinked polycarboxybetaine, a crosslinked polysulfobetaine, a crosslinked polyphosphobetaine, and a crosslinked polyphosphorylcholine.

11. The method of any one of the preceding Claims, wherein the zwitterionic microgel consists of a crosslinked zwitterionic polymer.

12. The method of any one of the preceding Claims, wherein the zwitterionic microgel comprises a crosslinked mixed charge copolymer having crosslinking range from about 0.01% to about 50%, such as from about 0.1% to about 30%, from about 0.1% to about 10%, or from about 1% to about 5%; and/or
wherein the zwitterionic microgel comprises a crosslinked mixed charge copolymer having covalent crosslinks, ionic crosslinks, or crosslinks formed by association of a portion of one mixed charge copolymer with another.

13. A method for cell culturing, the method comprising culturing a population of cells in a matrix comprising a zwitterionic microgel,
wherein the zwitterionic microgel comprises a microgel composition prepared from physical processing of a crosslinked zwitterionic hydrogel or a crosslinked mixed charge hydrogel to provide a microgel composition comprising a plurality of crosslinked zwitterionic or a plurality of crosslinked mixed charge microgel units, and
wherein the physical processing comprises cutting, chopping, grinding, grading, templating, rubbing, mincing, extruding, or crushing the crosslinked zwitterionic hydrogel or the crosslinked mixed charge hydrogel to provide the microgel composition.

14. The method according to claim 13, wherein the method comprises expanding the population of cells.

## Patentansprüche

1. Ein Verfahren zur schützenden Lagerung einer Population von Zellen, eines Gewebes oder eines Organs, das das Lagern einer Population von Zellen, eines Gewebes oder eines Organs in einem zwitterionischen Mikrogel umfasst, um gelagerte Zellen, ein gelagertes Gewebe oder ein gelagertes Organ bereitzustellen, wobei die gelagerten Zellen, das gelagerte Gewebe oder das gelagerte Organ ihre biologische Funktion bei der Lagerung im Wesentlichen beibehalten;
wobei das zwitterionische Mikrogel eine Mikrogelzusammenfassung umfasst, die aus der physikalischen Verarbeitung eines vernetzten zwitterionischen Hydrogels oder eines vernetzten Hydrogels mit gemischter Ladung hergestellt wird, um eine Mikrogelzusammenfassung bereitzustellen, die eine Vielzahl von vernetzten zwitterionischen oder eine Vielzahl von vernetzten Mikrogeleinheiten mit gemischter Ladung umfasst; und
wobei die physikalische Verarbeitung das Schneiden, Hacken, Mahlen, Klassifizieren, Schablonieren, Reiben, Zerkleinern, Extrudieren oder Zerquetschen des vernetzten zwitterionischen Hydrogels oder des vernetzten Hydrogels mit gemischter Ladung umfasst, um die Mikrogelzusammenfassung bereitzustellen.

2. Verfahren nach Anspruch 1, wobei die Zellen aus Folgenden ausgewählt sind: pluripotenten und multipotenten Stamm- und Progenitorzellen; hämatopoetischen Zellen; gentechnisch hergestellten Zellen, wie etwa Impfstoffen, Immunzellen und Progenitoren oder differenzierten Zelllinien davon, wie etwa T-Zellen, B-Zellen, dendritischen Zellen, antigenpräsentierenden Zellen; Pankreas-Inselzellen oder anderen insulinproduzierenden Zellen; Nervensystemzellen und -progenitoren; und Herz-Kreislauf-Systemzellen und -progenitoren.

3. Verfahren nach Anspruch 1 oder Anspruch 2, wobei das Gewebe Folgendes ist: Muskel, wie etwa Skelettmuskel, glatter Muskel, Herzmuskel, Gefäßmuskel einschließlich Blutgefäßen; Nervengewebe, wie etwa peripheres Nervengewebe, Zentralnervengewebe einschließlich Gewebe, das aus Neuroglia besteht, die Astrozyten, Mikrogliazellen, Ependymzellen, Oligodendrozyten, Satellitenzellen oder Schwannzellen sind; Bindegewebe, wie etwa Knorpel, elastischer Knorpel, Faserknorpel, Knochengewebe, weißes Fettgewebe, braunes Fettgewebe, Faszien, Blut; subkutanes Gewebe; oder Epithelgewebe, wie etwa Plattenepithel, kubisches Epithel, Säulenepithel, mehrschichtiges Epithel, pseudomehrschichtiges Epithel, Übergangsepithel.

4. Verfahren nach einem der vorhergehenden Ansprüche, wobei das Organ Folgendes ist: Niere; Herz; Gehirn einschließlich Großhirn, Großhirnhemisphären, Zwischenhim; Hirnstamm einschließlich Mittelhirn, Pons, Medulla oblongata, Cerebellum, Rückenmark, Ventrikelsystem, Plexus choroideus; Ösophagus; Pharynx; Speicheldrüsen, wie etwa Parotisdrüsen, Submandibularisdrüsen, Sublingualdrüsen; Magen; Dünndarm einschließlich Duodenum, Jejunum, Ileum; Dickdarm; Leber; Gallenblase; Bauchspeicheldrüse; Nase einschließlich Nasenhöhle, Pharynx, Larynx, Trachea, Bronchien, Lungen; Harnleiter; Blase; Harnröhre; Arterien; Venen; Kapillaren; Lymphgefäß; Lymphknoten; Knochenmark; Thymus; Milz; mit dem Darm assoziiertes Lymphgewebe, wie etwa Tonsillen; Auge; Ohr; olfaktorisches Epithel; Zunge; oder Haut.

5. Verfahren nach einem der vorhergehenden Ansprüche, wobei die Zellen, das Gewebe oder das Organ in Abwesenheit eines Kryoprotektivums oder in Gegenwart eines Osmolyten gelagert werden.

6. Verfahren nach einem der vorhergehenden Ansprüche, wobei die Zellen zur Verwendung mittels Filtration aus dem Mikrogel isoliert werden.

7. Verfahren nach einem der vorhergehenden Ansprüche, wobei das zwitterionische Mikrogel ein vernetztes zwitterionisches Polymer umfasst, das einen Vernetzungsbereich von etwa 0.005% bis etwa 100%, wie etwa 0.01% bis etwa 30% oder etwa 0.01% bis etwa 10% aufweist.

8. Verfahren nach einem der vorhergehenden Ansprüche, wobei das zwitterionische Mikrogel ein vernetztes zwitterionisches Polymer umfasst, das kovalente Vernetzungen, ionische Vernetzungen oder durch Assoziation eines Anteils eines zwitterionischen Polymers mit einem anderen gebildete Vernetzungen aufweist.

9. Verfahren nach einem der vorhergehenden Ansprüche, wobei das zwitterionische Mikrogel abbaubare Vernetzungen, wie etwa eine hydrolytische, proteolytische oder andere auf Stimuli ansprechende oder physiologisch ansprechende Gruppe, umfasst.

10. Verfahren nach einem der vorhergehenden Ansprüche, wobei das zwitterionische Mikrogel ein vernetztes zwitterionisches Polymer, das aus einem vernetzten Polycarboxybetain, einem vernetzten Polysulfobetain, einem vernetzten Polyphosphobetain und einem vernetzten Polyphosphorylcholin ausgewählt ist, umfasst.

11. Verfahren nach einem der vorhergehenden Ansprüche, wobei das zwitterionische Mikrogel aus einem vernetzten zwitterionischen Polymer besteht.

12. Verfahren nach einem der vorhergehenden Ansprüche, wobei das zwitterionische Mikrogel ein vernetztes Copolymer mit gemischter Ladung umfasst, das einen Vernetzungsbereich von etwa 0.01% bis etwa 50%, wie etwa von etwa 0.1% bis etwa 30%, von etwa 0.1% bis etwa 10% oder von etwa 1% bis etwa 5% aufweist; und/oder
wobei das zwitterionische Mikrogel ein vernetztes Copolymer mit gemischter Ladung umfasst, das kovalente Vernetzungen, ionische Vernetzungen oder durch Assoziation eines Anteils eines Copolymers mit gemischter Ladung mit einem anderen gebildete Vernetzungen aufweist.

13. Ein Verfahren zum Kultivieren von Zellen, wobei das Verfahren das Kultivieren einer Population von Zellen in einer Matrix umfasst, die ein zwitterionisches Mikrogel umfasst,
wobei das zwitterionische Mikrogel eine Mikrogelzusammenfassung umfasst, die aus einer physikalischen Verarbeitung eines vernetzten zwitterionischen Hydrogels oder eines vernetzten Hydrogels mit gemischter Ladung hergestellt wird, um eine Mikrogelzusammenfassung bereitzustellen, die eine Vielzahl von vernetzten zwitterionischen oder eine Vielzahl von vernetzten Mikrogeleinheiten mit gemischter Ladung umfasst, und
wobei die physikalische Verarbeitung das Schneiden, Hacken, Mahlen, Klassifizieren, Schablonieren, Reiben, Zerkleinern, Extrudieren oder Zerquetschen des vernetzten zwitterionischen Hydrogels oder des vernetzten Hydrogels mit gemischter Ladung umfasst, um die Mikrogelzusammenfassung bereitzustellen.

14. Verfahren nach Anspruch 13, wobei das Verfahren das Expandieren der Population von Zellen umfasst.

## Revendications

1. Procédé de stockage de manière à les protéger d'une population de cellules, d'un tissu ou d'un organe, comprenant le stockage d'une population de cellules, d'un tissu ou d'un organe dans un microgel zwittérionique pour produire des cellules stockées, un tissu stocké ou un organe stocké, dans lequel les cellules stockées, le tissu stocké ou l'organe stocké conservent sensiblement leur fonction biologique lors du stockage;
dans lequel le microgel zwittérionique comprend une composition de microgel préparée par traitement physique d'un hydrogel zwittérionique réticulé ou d'un hydrogel à charge mixte réticulé pour produire une composition de microgel comprenant une pluralité d'unités de microgel zwittérionique réticulé ou une pluralité d'unités de microgel à charge mixte réticulé; et
dans lequel le traitement physique comprend le découpage, le hachage, le broyage, le criblage, le modelage au gabarit, le frottement, le coupage en morceaux, l'extrusion ou la fragmentation de l'hydrogel zwittérionique réticulé ou de l'hydrogel à charge mixte réticulé pour produire la composition de microgel.

2. Procédé selon la revendication 1, dans lequel les cellules sont sélectionnées parmi des cellules souches et des cellules progénitrices pluripotentes et multipotentes; des cellules hématopoïétiques; des cellules génétiquement modifiées, telles que des vaccins, des cellules immunitaires et des progéniteurs ou des lignées différenciées de ceux-ci telles que des cellules T, des cellules B, des cellules dendritiques, des cellules présentatrices d'antigènes; des îlots pancréatiques ou d'autres cellules productrices d'insuline; des cellules et des progéniteurs du système nerveux; et des cellules et des progéniteurs du système cardiovasculaire.

3. Procédé selon la revendication 1 ou la revendication 2, dans lequel le tissu est un muscle, tel qu'un tissu squelettique, lisse, cardiaque, vasculaire y compris des vaisseaux sanguins; un tissu nerveux, tel qu'un tissu nerveux périphérique, un tissu nerveux central y compris un tissu constitué de névroglies qui sont des astrocytes, des cellules microgliales, des cellules épendymaires, des oligodendrocytes, des cellules satellites ou des cellules de Schwann; un tissu conjonctif, tel qu'un cartilage, un cartilage élastique, un fibrocartilage, un tissu osseux, un tissu adipeux blanc, un tissu adipeux brun, un fascia, du sang; un tissu sous-cutané; ou un tissu épithélial, tel qu'un épithélium squameux, un épithélium cubique, un épithélium colonnaire, un épithélium stratifié, un épithélium pseudostratifié, un épithélium transitionnel.

4. Procédé selon l'une quelconque des revendications précédentes, dans lequel l'organe est un rein; un coeur; un cerveau, y compris un télencéphale, des hémisphères cérébraux, un diencéphale; un tronc cérébral, y compris un mésencéphale, un pont, de la moelle allongée, un cervelet, de la moelle épinière, un système ventriculaire, un plexus choroïde; un oesophage; un pharynx; des glandes salivaires, telles que des glandes parotides, des glandes sous-mandibulaires, des glandes sublinguales; un estomac; un intestin grêle, y compris un duodénum, un jéjunum, un iléon; un gros intestin; un foie; une vésicule biliaire; un pancréas; un nez, y compris une cavité nasale, un pharynx, un larynx, une trachée, des bronches, des poumons; des uretères; une vessie; un urètre; des artères; des veines; des capillaires; un vaisseau lymphatique; un ganglion lymphatique; de la moelle osseuse; un thymus; une rate; un tissu lymphoïde associé à l'intestin, tel que des amygdales; un oeil; une oreille; un épithélium olfactif; une langue; ou de la peau.

5. Procédé selon l'une quelconque des revendications précédentes, dans lequel les cellules, le tissu ou l'organe sont stockés en l'absence de cryoprotecteur ou en présence d'un osmolyte.

6. Procédé selon l'une quelconque des revendications précédentes, dans lequel les cellules sont isolées pour une utilisation par filtration à partir du microgel.

7. Procédé selon l'une quelconque des revendications précédentes, dans lequel le microgel zwittérionique comprend un polymère zwittérionique réticulé ayant une plage de réticulation d'environ 0.005 % à environ 100 %, par exemple d'environ 0.01 % à environ 30 %, ou d'environ 0.01 % à environ 10 %.

8. Procédé selon l'une quelconque des revendications précédentes, dans lequel le microgel zwittérionique comprend un polymère zwittérionique réticulé comportant des réticulations covalentes, des réticulations ioniques ou des réticulations formées par association d'une partie d'un polymère zwittérionique avec un autre.

9. Procédé selon l'une quelconque des revendications précédentes, dans lequel le microgel zwittérionique comprend des réticulations dégradables, telles qu'un groupe hydrolytique, protéolytique, ou un autre groupe sensible aux stimuli ou physiologiquement réactif.

10. Procédé selon l'une quelconque des revendications précédentes, dans lequel le microgel zwittérionique comprend un polymère zwittérionique réticulé sélectionné parmi une polycarboxybétaïne réticulée, une polysulfobétaïne réticulée, une polyphosphobétaïne réticulée et une polyphosphorylcholine réticulée.

11. Procédé selon l'une quelconque des revendications précédentes, dans lequel le microgel zwittérionique est constitué d'un polymère zwittérionique réticulé.

12. Procédé selon l'une quelconque des revendications précédentes, dans lequel le microgel zwittérionique comprend un copolymère à charge mixte réticulé ayant une plage de réticulation d'environ 0.01 % à environ 50 %, par exemple d'environ 0.1 % à environ 30 %, d'environ 0.1 % à environ 10 %, ou d'environ 1 % à environ 5 %; et/ou
dans lequel le microgel zwittérionique comprend un copolymère à charge mixte réticulé comportant des réticulations covalentes, des réticulations ioniques, ou des réticulations formées par association d'une partie d'un copolymère à charge mixte avec un autre.

13. Procédé de culture cellulaire, le procédé comprenant la culture d'une population de cellules dans une matrice comprenant un microgel zwittérionique,
dans lequel le microgel zwittérionique comprend une composition de microgel préparée par traitement physique d'un hydrogel zwittérionique réticulé ou d'un hydrogel à charge mixte réticulé pour produire une composition de microgel comprenant une pluralité d'unités de microgel zwittérionique réticulé ou une pluralité d'unités de microgel à charge mixte réticulé; et
dans lequel le traitement physique comprend le découpage, le hachage, le broyage, le criblage, le modelage au gabarit, le frottement, le coupage en morceaux, l'extrusion ou la fragmentation de l'hydrogel zwittérionique réticulé ou de l'hydrogel à charge mixte réticulé pour produire la composition de microgel.

14. Procédé selon la revendication 13, le procédé comprenant l'expansion de la population de cellules.
